# EUROPEAN PATENT SPECIFICATION

(11) **EP 2 379 074 B1**
(45) Date of publication and mention of the grant of the patent: **13.11.2013**
(21) Application number: 09801957.3
(22) Date of filing: 10.12.2009
(51) Int. Cl.: C07K 5/08, A61K 31/395

(54) **MACROLACTONE DERIVATIVES, METHOD FOR THE PRODUCTION THEREOF AND USE THEREOF FOR THE TREATMENT OF CANCER**
MAKROLAKTON-DERIVATE, HERSTELLUNGSVERFAHREN DAFÜR UND VERWENDUNG DAVON ZUR BEHANDLUNG VON KREBS
DÉRIVÉS DE MACROLACTONE, PROCÉDÉ POUR LEUR PRODUCTION ET LEUR UTILISATION POUR LE TRAITEMENT DU CANCER

(30) Priority: 17.12.2008 EP 08291202
(43) Date of publication of application: 26.10.2011
(73) Proprietor: SANOFI, 75008 Paris (FR)
(72) Inventor: HOFFMANN, Holger, 65926 Frankfurt am Main (DE); CASPERS, Michael, 65926 Frankfurt am Main (DE); SCHUMMER, Dietmar, 65926 Frankfurt am Main (DE); KOGLER, Herbert, 65926 Frankfurt am Main (DE); KLEMKE-JAHN, Christine, 65926 Frankfurt am Main (DE)
(74) Representative: Kujath, Eckard
(86) International application number: PCT/EP2009/066805
(87) International publication number: WO 2010/069850

(56) References cited:
- WO-A-2006/100330
- WO-A-2009/003595

## Description

Cancer is a disease of humans and animals which is for the most part fatal and which is caused by the uncontrolled growth of endogenous cells. Cancer is the term for the formation of malignant tumors (malignomas) and of neoplasm (tumors or carcinomas) or for the malignant degeneration and disturbed maturation of white blood cells (leukemia, blood cancer). Cancer cells or tumor cells arise as the result of the transformation of endogenous cells. The malignancy of the cancer cell is expressed in the autonomy of its growth that is in the ability of the cell to grow in an uninhibited manner and without being fitted into the structure of the organs and also to grow in an infiltrating manner, thereby destroying tissue. The formation of disseminations (metastases) at a distance from the tumor, after tumor cells have been spread by way of the blood or the lymph, is a sure sign of malignancy. Cancer is one of the most frequent causes of death in humans and there is therefore a great need for methods and means for curing or treating malignant degenerations.

Aside from the, if possible radical, surgical removal of the tumor, the possibilities for treating malignant tumors include radiological therapy using X-rays, α-rays, β-rays and γ-rays, immunotherapy and chemotherapy. At present, immunotherapy can only be used to a limited extent. The chemotherapy of tumors is understood as meaning the administration of cell poisons (cytostatic agents) for treating tumors and tumor cells which remain, usually following local surgical treatment or irradiation. These substances interfere specifically in certain processes in cell division, which means that tissues containing a high proportion of dividing cells, such as rapidly growing tumor tissues, react more sensitively. The cytostatic agents which are used are alkylating compounds, such as cyclophosphamide, antimetabolites, such as methotrexate, alkaloids, such as vincristine, and antibiotics, such as daunomycin or adriamycin. However, due to massive side-effects, all these agents suffer from severe disadvantages, such that the death of the affected patient is only delayed and not averted. Furthermore, the degenerate (cancer) cells develop resistances to the agents which are used; while the medicaments which are being used at the time then no longer have any cytostatic effect, they are still toxic as a consequence of the side-effects. In addition, it has been found that the efficacy achieved by using cytostatic agents in combination or in sequence exceeds that achieved by using a single cytostatic agent (monotherapy) and, as a result, it is possible that the substantial side-effects are not additive in connection with polychemotherapy. For all these reasons, novel chemotherapeutic agents are urgently required and are therefore being sought world-wide.

International patent application no. PCT/EP2008/004971 (WO 2009/003595) describes macrocycles of the formula 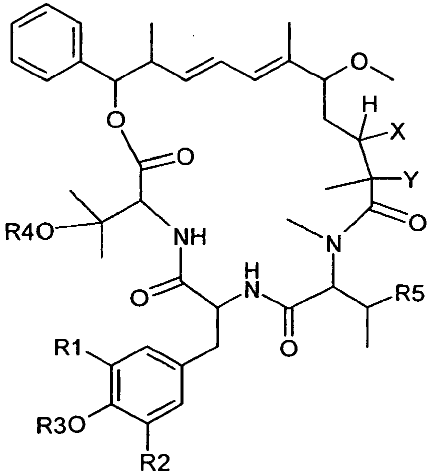 wherein
X and Y independently of one another are OH, O-(C₁-C₆)-alkyl, NH₂ or NH-(C₁-C₆)-alkyl, or X and Y together form a group -O-;
R1 and R2 independently of one another are H or Cl;
R3 is H, (C₁-C₆)-alkyl, C(=O)-(C₁-C₆)-alkyl or (C₁-C₆)-alkylene-NH-(C₁-C₆)-alkyl; R4 is H, (C₁-C₆)-alkyl or C(=O)-(C₁-C₆)-alkyl; and
R5 is ethyl;
or a physiologically tolerable salt of the said compound.

The compounds described in PCT/EP2008/004971 are obtainable by fermenting the microorganism strain *Nannocystis sp.* ST 201196 (DSM 18870) and optional further derivatization, and are useful for the treatment and/or prophylaxis of fungal disorders.

The present invention relates to a compound of the formula (I), 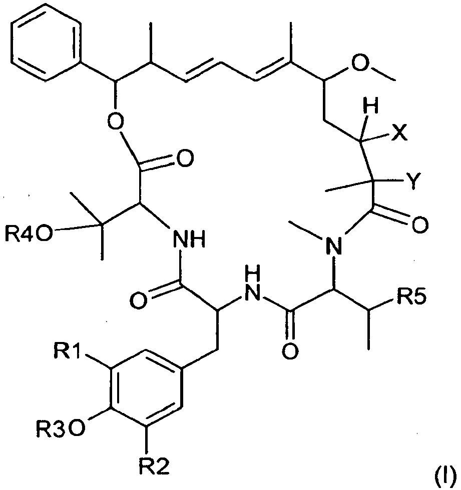 wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached;
R1 and R2 independently of one another are H, Cl or Br;
R3 is H, (C₁-C₆)-alkyl, C(=O)-(C₁-C₆)-alkyl or (C₁-C₆)-alkylene-NH-(C₁-C₆)-alkyl;
R4 is H, (C₁-C₆)-alkyl or C(=O)-(C₁-C₆)-alkyl; and
R5 is methyl or ethyl;
or a physiologically tolerable salt of a compound of the formula (I),
for use in the treatment and/or prophylaxis of cancer diseases.

Additionally, the present invention relates to the use of a compound of the formula (I) for the preparation of a medicament for the treatment and/or prophylaxis of cancer diseases.

X and Y preferably together form a further bond between the C atoms to which they are attached, consequently resulting in a double bond between the said C atoms, or X and Y together form a group -O-, consequently X and Y in the corresponding preferred compound, together with the C atoms to which they are bonded, form an epoxide group. More preferred, X and Y together form a group -O-.

Preferably, at least one of R1 and R2 is Cl or Br. More preferred, R1 and R2 are both Cl.

R3 is preferably H or (C₁-C₆)-alkyl. More preferred, R3 is H or methyl.

R4 is preferably H.

R5 is preferably ethyl.

Particularly preferably, the invention relates to a compound of the formula (I), wherein X and Y together form a group -O-, or X and Y form a further double bond between the C atoms to which they are attached, R1 and R2 independently of one another are H, Cl or Br, R3 and R4 independently of one another are H, (C₁-C₆)-alkyl or C(=O)-(C₁-C₆)-alkyl, and R5 is methyl or ethyl.

More preferably, the invention relates to a compound of the formula (I), wherein X and Y together form a group -O-, or X and Y form a further double bond between the C atoms to which they are attached, R1 and R2 independently of one another are H, Cl or Br, R3 is H or (C₁-C₆)-alkyl, R4 is H, and R5 is methyl or ethyl.

Most preferably, the invention relates to a compound of the formula (I), wherein X and Y together form a group -O-, or X and Y form a further double bond between the C atoms to which they are attached, R1 and R2 are both Cl, R3 is H or (C₁-C₆)-alkyl, preferably H or methyl, R4 is H, and R5 is methyl or ethyl.

(C₁-C₆)-Alkyl is a straight-chain or branched alkyl group having 1 to 6 carbon atoms, for example methyl, ethyl, n-propyl, isopropyl, n-butyl, sec-butyl, tert-butyl, n-pentyl or n-hexyl.

Examples of the compound of the formula (I) are as follows:

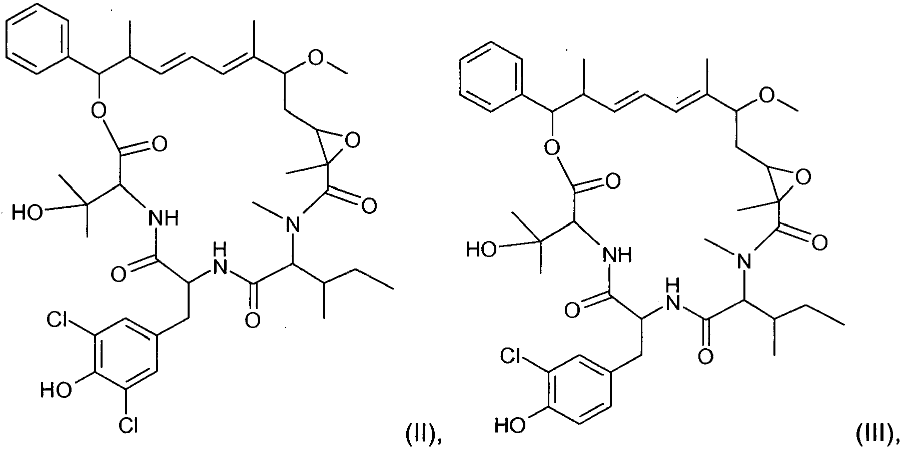

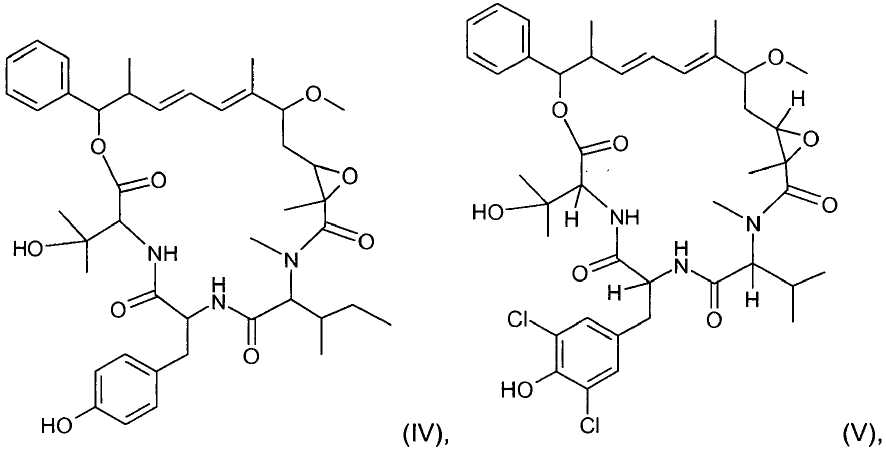

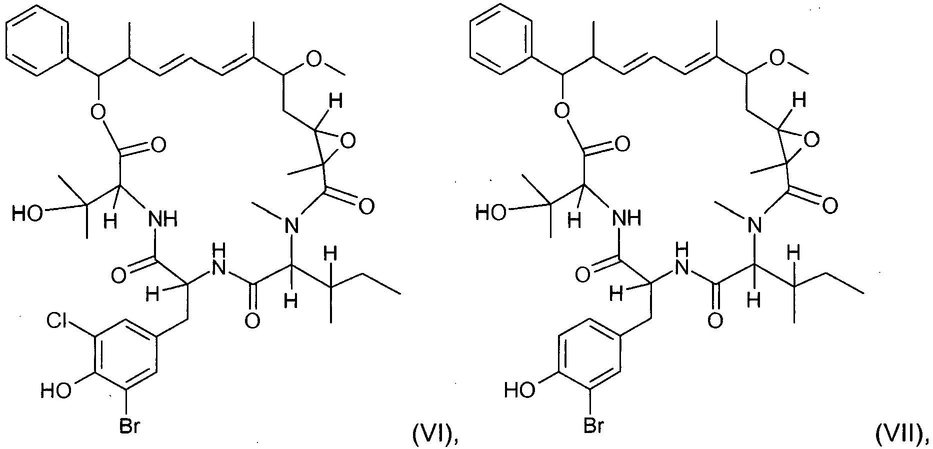

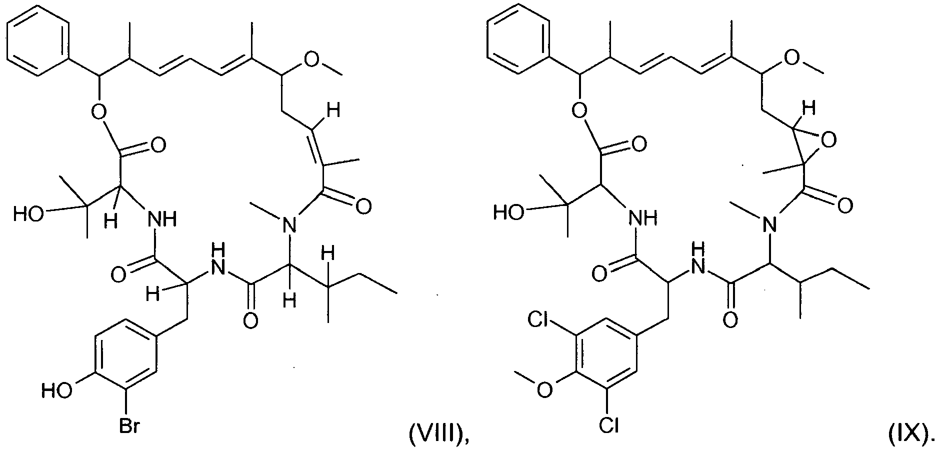

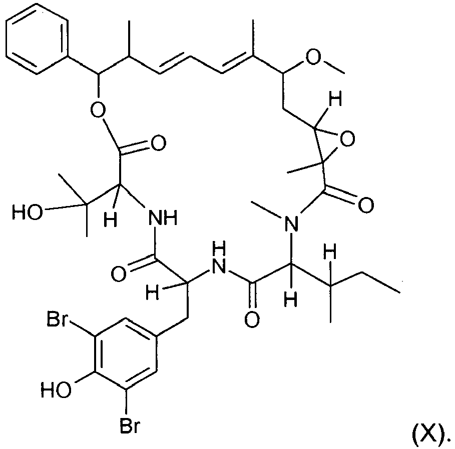

The present invention further relates to a compound of the formula (I), 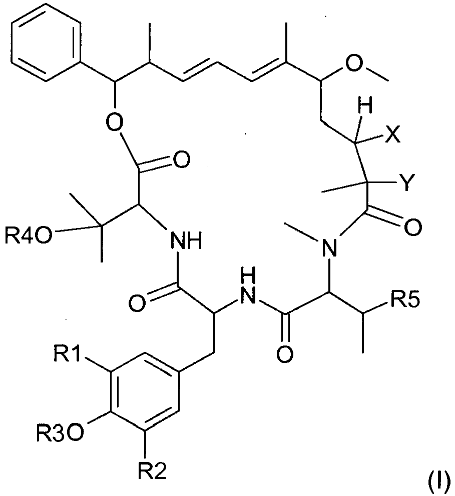 wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached,
R1 and R2 independently of one another are H, Cl or Br,
R3 is H, (C₁-C₆)-alkyl, C(=O)-(C₁-C₆)-alkyl or (C₁-C₆)-alkylene-NH-(C₁-C₆)-alkyl,
R4 is H, (C₁-C₆)-alkyl or C(=O)-(C₁-C₆)-alkyl, and
R5 is methyl or ethyl,
provided that when
X and Y independently of one another are OH, O-(C₁-C₆)-alkyl, NH₂ or NH-(C₁-C₆)-alkyl, or X and Y together form a group -O-,
R1 and R2 independently of one another are H or Cl,
R3 is H, (C₁-C₆)-alkyl, C(=O)-(C₁-C₆)-alkyl or (C₁-C₆)-alkylene-NH-(C₁-C₆)-alkyl, and
R4 is H, (C₁-C₆)-alkyl or C(=O)-(C₁-C₆)-alkyl,
R5 may not be ethyl,
or a physiologically tolerable salt of a compound of the formula (I).

Examples of the compound of the formula (I) are the compounds of the formulae (V), (VI), (VII) and (VIII), *vide supra.*

Chiral centers in the compounds of the formulae (I) can be present, if not stated otherwise, in the R or in the S configuration. The invention relates both to the optically pure compounds and stereoisomer mixtures like enantiomer mixtures and diastereomer mixtures.

Physiologically tolerable salts of compounds of the formulae (I) are understood as meaning both their organic and inorganic salts, as are described in Remington's Pharmaceutical Sciences (17th edition, page 1418 (1985)). On account of the physical and chemical stability and the solubility, sodium, potassium, calcium and ammonium salts, inter alia, are preferred for acidic groups; salts of hydrochloric acid, sulfuric acid, phosphoric acid or of carboxylic acids or sulfonic acids, such as, for example, acetic acid, citric acid, benzoic acid, maleic acid, fumaric acid, tartaric acid and p-toluenesulfonic acid, inter alia, are preferred for basic groups.

The present invention furthermore relates to all obvious chemical equivalents of the compounds of the formulae (I) and the use of obvious chemical equivalents of the compounds of the formulae (I). Equivalents of this type are compounds which exhibit a slight chemical difference, thus have the same action or are converted to the compounds according to the invention under mild conditions. The equivalents mentioned also include, for example, salts, reduction products, oxidation products, esters, ethers, acetals or amides of the compounds of the formula (I) and equivalents which the person skilled in the art can prepare using standard methods.

The compounds of the formula (I) can generally be prepared as described in International patent application no. PCT/EP2008/004971.

The invention furthermore relates to a process for the preparation of a compound of the formula (I) 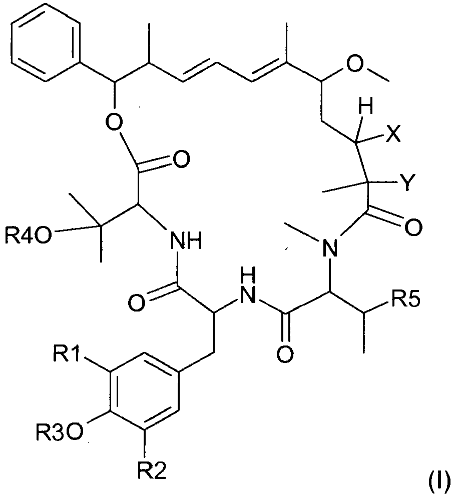 wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached,
R1 and R2 independently of one another are H, Cl or Br,
R3 is H, (C₁-C₆)-alkyl, C(=O)-(C₁-C₆)-alkyl or (C₁-C₆)-alkylene-NH-(C₁-C₆)-alkyl,
R4 is H, (C₁-C₆)-alkyl or C(=O)-(C₁-C₆)-alkyl, and
R5 is methyl or ethyl,
provided that when
X and Y independently of one another are OH, O-(C₁-C₆)-alkyl, NH₂ or NH-(C₁-C₆)-alkyl, or X and Y together form a group -O-,
R1 and R2 independently of one another are H or Cl,
R3 is H, (C₁-C₆)-alkyl, C(=O)-(C₁-C₆)-alkyl or (C₁-C₆)-alkylene-NH-(C₁-C₆)-alkyl, and
R4 is H, (C₁-C₆)-alkyl or C(=O)-(C₁-C₆)-alkyl,
R5 may not be ethyl,
or a physiologically tolerable salt of a compound of the formula (I),
which comprises
1. fermenting the strain *Nannocystis sp.* ST 201196 (DSM 18870) or one of its variants and/or mutants under suitable conditions in a culture medium which contains a Cl and/or a Br source, until one or more of the compounds of the formula (I) accumulates in the culture medium and
2. isolating a compound of the formula (I) from the culture medium, and
3. optionally derivatizing the compound of the formula (I) and/or converting it into a physiologically tolerable salt.

Preferably, at least one Br source is present in the culture medium.

An isolate of the microorganism strain *Nannocystis sp.* ST 201196 was deposited at the Deutsche Sammlung von Mikroorganismen und Zellkulturen [German Collection of Microorganisms and Cell Cultures] GmbH (DSMZ), Mascheroder Weg 1 B, 38124 Braunschweig, Germany, according to the rules of the Budapest Convention on the 09.11.2003 under the following number. The following number was assigned as the deposit number: DSM 18870.

The vegetative cells of the strain *Nannocystis sp.* ST 201196 (DSM 18870) have a characteristic rod shape. On solid nutrient media, *Nannocystis sp.* ST 201196 (DSM 18870) forms orange-yellow fruiting bodies, which contain round myxospores. The taxonomy of the strain ST 201196 can therefore be described as Myxobacterium sp..

Instead of the strain *Nannocystis sp.* ST 201196 (DSM 18870), it is also possible to employ its mutants and/or variants which synthesize one or more of the compounds according to the invention.

A mutant is a microorganism in which one or more genes of the genome have been modified, where the gene or the genes which are responsible for the ability of the organism to produce the inventive compound remain functional and inheritable.

Mutants of this type can be produced in a manner known per se by physical means, for example irradiation, such as with ultraviolet or X-ray beams, or chemical mutagens, such as, for example, ethyl methanesulfonate (EMS); 2-hydroxy-4-methoxybenzophenone (MOB) or N-methyl-N'-nitro-N-nitrosoguanidine (MNNG), or as described by Brock et al. in "Biology of Microorganisms", Prentice Hall, page 304-315 (1984).

A variant is a phenotype of the microorganism. Microorganisms have the ability to adapt to their environment and therefore show marked physiological flexibility. In the case of phenotypic adaptation, all cells of the microorganism are involved, where the nature of the modification is not genetically conditioned and is reversible under modified conditions (H. Stolp, Microbial ecology: organisms, habitats, activities. Cambridge University Press, Cambridge, GB, page 180, 1988).

Screening for mutants and/or variants which synthesize one or more of the compounds according to the invention is carried out according to the following scheme:
- lyophilization of the fermentation medium
- extraction of the lyophilizate with an organic solvent
- extraction of the compound from the culture filtrate using solid phases
- analysis by means of HPLC, TLC or by testing of the biological activity.

The described fermentation conditions apply for *Nannocystis sp.* ST 201196 (DSM 18870) and for mutants and/or variants thereof.

The culture medium is a nutrient solution or a solid medium containing at least one carbon and nitrogen source, and the customary inorganic salts.

The Cl source used can be, for example, NaCl or CaCl₂. In this case, the strain *Nannocystis sp.* ST 201196 (DSM 18870) preferably produces compounds of the formula (I), in which both radicals R1 and R2 are equal to Cl or in which R1 is equal to Cl and R2 is equal to H. Preferably, the invention relates to a process for the preparation of a compound of the formula (I), where R1 and R2 are equal to Cl. Furthermore, the invention preferably relates to a process for the preparation of a compound of the formula (I), where R1 is equal to H, R2 is equal to Cl.

The feeding of the strain *Nannocystis sp.* ST201196 (DSM 18870) with CaBr₂ gave three new bromo-containing macrolactons. The Br source used can be an alkali or alkaline bromide, for example, NaBr or CaBr₂. In this case, the strain *Nannocystis sp.* ST 201196 (DSM 18870) preferably produces compounds of the formula (I), in which R1 and R2 are independently of each other H, Cl or Br, wherein at least one of R1 or R2 is Br, preferably R1 is Cl and R2 is Br or in which R1 is Br and R2 is H. The process according to the invention can be employed for fermentation on the laboratory scale (milliliter to liter scale) and for the industrial scale (cubic meter scale).

Suitable carbon sources for the fermentation are assimilable carbohydrates and sugar alcohols, such as glucose, lactose, sucrose, or D-mannitol and carbohydrate-containing natural products, such as, for example, malt extract or yeast extract. Suitable nitrogen-containing nutrients are: amino acids; synthetically or biosynthetically peptides and proteins and their degradation products, for example Probion F (Applied Microbiology and Biotechnology 1984, 19(1), 23-28), casein, peptone or tryptone; meat extracts; yeast extracts; gluten; ground seeds, for example of corn, wheat, beans, soybeans or of the cotton plant; distillation residues from alcohol production; meat meals; yeast extracts; ammonium salts; nitrates. Inorganic salts are, for example, chlorides, carbonates, sulfates or phosphates of the alkali metals or alkaline earth ametals, iron, zinc, cobalt and manganese. Trace elements are, for example, cobalt and manganese.

Suitable conditions for the formation of the Cl-containing substances according to the invention are as follows: The formation of the substances according to the invention preferably proceeds in a culture medium which contains 0.05 to 5%, preferably 0.1 to 2.5%, of soybean meal; 0.05 to 5%, preferably 0.2 to 2% of soluble starch; 0.02 to 2%, preferably 0.05 to 0.5% of glucose; 0.02 to 2%, preferably 0.2 to 0.8% of HEPES; 0.02 to 1.0%, preferably 0.05 to 0.5%, of CaCl₂ x 2 H₂O; 0.02 to 1.5%, preferably 0.05 to 0.7%, of MgSO₄ x 7 H₂O, 0.00001 % to 0.001 % of cyanocobalamin and 1-5% of the adsorber resin XAD-16. The data in percent are in each case based on the weight of the entire nutrient solution.

Suitable conditions for the formation of the Br-containing substances according to the invention are as follows: The formation of the substances according to the invention preferably proceeds in a culture medium which contains 0.05 to 5%, preferably 0.1 to 2.5%, of soybean meal; 0.05 to 5%, preferably 0.2 to 2% of soluble starch; 0.02 to 2%, preferably 0.05 to 0.5% of glucose; 0.02 to 2%, preferably 0.2 to 0.8% of HEPES; 0.02 to 1.0%, preferably 0.05 to 0.5%, of CaBr₂ · xH₂O; 0.02 to 1.5%, preferably 0.05 to 0.7%, of MgSO₄ x 7 H₂O, 0.00001 % to 0.001 % of cyanocobalamin and 1-5% of the adsorber resin XAD-16. The data in percent are in each case based on the weight of the entire nutrient solution.

The culturing of the microorganism is carried out aerobically, that is, for example, in submerse form with shaking or stirring in shaker flasks or fermenters or on a solid medium, optionally with the introduction of air or oxygen. It can be carried out in a temperature range from approximately 18 to 35°C, preferably at approximately 20 to 32°C, in particular at 27 to 30°C. The pH range should be between 4 and 10, preferably between 6.5 and 9. The microorganism is in general cultured under these conditions over a period of 3 to 18 days, preferably 144 to 216 hours. Advantageously, culturing is carried out in a number of stages, i.e. first one or more pre-cultures is prepared in a liquid nutrient medium, which is then inoculated into the actual production medium, the main culture, for example in the volume ratio 1:10 to 1:100. The pre-culture is obtained, for example, by inoculating the strain in the form of vegetative cells or fruiting bodies into a nutrient solution and allowing it to grow for approximately 3 to 13 days, preferably 96 to 240 hours. Vegetative cells and/or fruiting bodies can be obtained, for example, by allowing the strain to grow for approximately 3 to 15 days, preferably 7 to 10 days, on a solid or liquid nutrient medium, for example yeast agar.

The isolation or purification of the substances of the formula (I) from the culture medium is carried out according to known methods taking into consideration the chemical, physical and biological properties of the natural substances. HPLC was used for testing the concentration of the respective derivatives in the culture medium or in the individual isolation stages.

For isolation, the culture broth is centrifuged and/or filtered off through a suction filter. The mycelium is lyophilized with the XAD, subsequently the natural substances are extracted from the lyophilizate using an organic solvent, for example methanol or 2-propanol. The organic solvent phase contains the natural substances according to the invention; it is optionally concentrated in vacuo and further purified by liquid-liquid extraction using water and an organic solvent, for example dichloromethane, ethyl acetate or Isobutyl methyl ketone.

The further purification of one or more compounds according to the invention is carried out by chromatography on suitable materials, preferably, for example, on molecular sieves, on silica gel, alumina, on ion exchangers or on adsorber resins or on reversed phases (RP). With the aid of this chromatography, the natural substance derivatives are separated. The chromatography of the compounds according to the invention is carried out using buffered aqueous solutions or mixtures of aqueous and organic solutions.

Mixtures of aqueous or organic solutions are understood as meaning all organic solvents miscible with water, preferably methanol, 2-propanol and acetonitrile, in a concentration of 0 to 100% of solvent or alternatively all buffered aqueous solutions which are miscible with organic solvents. The buffers to be used are the same as indicated above.

The separation of the compounds according to the invention on the basis of their differing polarity is carried out with the aid of reversed phase chromatography, for example on MCI® (adsorber resin, Mitsubishi, Japan) or Amberlite XAD® (TOSOHAAS), or on another hydrophobic material, such as, for example, on RP-8 or RP-18 phases. Moreover, the separation can be carried out with the aid of normal phase chromatography, for example on silica gel, alumina and the like.

The chromatography of the natural substance derivatives was carried out according to methods known to the person skilled in the art, preferably using buffered, basic or acidified aqueous solutions or mixtures of aqueous solutions with alcohols or other, water-miscible organic solvents. Acetonitrile and/or methanol is preferably used as an organic solvent.

Buffered, basic or acidified aqueous solutions are understood as meaning, for example, water, phosphate buffer, ammonium acetate, ammonium formate, citrate buffer in a concentration of up to 0.5 M, and formic acid, acetic acid, trifluoroacetic acid, ammonia, triethylamine or all commercially available acids and bases known to the person skilled in the art, preferably in a concentration of up to 1 %. In the case of buffered aqueous solutions, 0.1 % ammonium acetate is particularly preferred.

Chromatography was carried out, for example, using a gradient which began with 100% water and ended with 100% solvent; a linear gradient of 5 to 95% acetonitrile was preferably operated.

Alternatively, gel chromatography or chromatography on hydrophobic phases can also be carried out. Gel chromatography is carried out on polyacrylamide or mixed polymer gels, such as, for example, Biogel-P 2® (Biorad) or Fractogel TSK HW 40® (Merck, Germany). The sequence of the aforementioned chromatographies is reversible.

If the compound of the formula (I) is present as a stereoisomer mixture, the stereoisomers can be separated by means of known methods, for example by separation by means of a chiral column.

The derivatization of the OH groups on the 3,5-dichlorotyrosine amino acid of the compound of the formula (I) [R3 equal to H] to an acyl group [R3 equal to C(=O)-(C₁-C₆)-alkyl)] and/or of the OH group on the 3-hydroxyvaline amino acid of the compound of the formula (I) [R4 equal to H] to an acyl group [R4 equal to C(=O)-(C₁-C₆)-alkyl)] is carried out according to methods known per se (J. March, Advanced Organic Chemistry, John Wiley & Sons, 6th Edition, 2007), for example by reaction with an acid chloride in the presence of a base or with an acid anhydride.

The alkylation of the OH group on the 3,5-dichlorotyrosine amino acid of the compound of the formula (I) [R3 equal to H] with an alkyl group [R3 equal to (C₁-C₆)-alkyl] and/or the OH group on the 3-hydroxyvaline amino acid of the compound of the formula (I) [R4 equal to H] with an alkyl group [R4 equal to (C₁-C₆)-alkyl] is carried out by means of methods known per se to the person skilled in the art (J. March, Advanced Organic Chemistry, John Wiley & Sons, 4th Edition, 1992), for example by reaction with a (C₁-C₆)-alkyl bromide in the presence of a base or in the case of a methylation by reaction with methyl iodide or Me₂SO4.

A selective differentiation between the phenolic OH group (R3=H) and the aliphatic OH group (R4=H) is carried out by means of methods known per se to the person skilled in the art for the introduction of protective groups (T. W. Greene, P. G. M. Wuts, Protective Groups in Organic Synthesis, John Wiley & Sons, 3rd Edition, 1999). For example, Pettus et al. (J. Am. Chem. Soc. 2000, 122, 6160-6168) describes a selective alkylation of a phenolic OH group in the presence of a tertiary aliphatic alcohol by reaction with (C₁-C₆)-alkyl bromide in the presence of K₂CO₃ in acetone or by reaction with (C₁-C₆)-alkyl-OH in the presence of (CF₃CO)₂O and CuCl₂ hydrate in DBU. A further possibility for differentiation between the phenolic OH group and the aliphatic OH group is carried out by means of the methods known per se to the person skilled in the art for the selective deprotection of a bis-alkylated [R3 equal to R4 equal to (C₁-C₆)-alkyl] or of a bis-acylated [R3 equal to R4 equal to C(=O)-(C₁-C₆)-alkyl)] compound of the formula (I) (T. W. Greene, P. G. M. Wuts, Protective Groups in Organic Synthesis, John Wiley & Sons, 4th Edition, 2006). For example, Jones et al. (J. Org. Chem. 2001, 66, 3688-3695) describe the selective deprotection of a tert-butylsilyl (TBS)-protected phenol in the presence of a TBS-protected tertiary alcohol by tert-butylammonium fluoride (TBAF) at -20°C. The phenolic OH group (R3 = H) can furthermore be derivatized to a group -(C₁-C₆)-alkylene-NH-(C₁-C₆)-alkyl by reaction with H₂N-(C₁-C₆)-alkyl in the presence of Cl-[(C₁-C₆)-alkyl]-Cl or Br-[(C₁-C₆)-alkyl]-Br.

The derivatization of compounds of the formula (I), in which X and Y form a group -O-to a compound of the formula (I), in which X and Y independently of one another are OH, O-(C₁-C₆)-alkyl, NH₂ or NH-(C₁-C₆)-alkyl, is carried out by means of methods known per se to the person skilled in the art (J. March, Advanced Organic Chemistry, John Wiley & Sons, 4th Edition, 1992), for example by reaction of the epoxide group with a (C₁-C₆)-alcoholate [X equal to OH if Y equal to O-(C₁-C₆)-alkyl, or Y equal to OH if X equal to O-(C₁-C₆)-alkyl], NH₃ [X equal to OH if Y equal to NH₂, or Y equal to OH if X equal to NH₂], or H₂N-(C₁-C₆)-alkyl [X equal to OH if Y equal to NH-(C₁-C₆)-alkyl, or Y equal to OH if X equal to NH-(C₁-C₆)-alkyl].

As shown in Tables 1, 2 and 3, the anti-tumor activity of the compounds of the formula (I) was determined by measuring the inhibition of the cell proliferation of various tumor cell lines:

| Table 1: In-vitro activity of compound (II) against various cancer cell lines | | |
|---|---|---|
| Cancer type | Cell line | IC₅₀ (nM) |
| Leukemia | HL60 | 9,5-15,0 |
| | CEM | 7,1-10,5 |
| Colon | HCT116 | 0,6-5,6 |
| | HT29 | 3,0-12,5 |
| Lung | H460 | 9,1-10,4 |
| Melanoma | B16F10 | 4,5-25,2 |
| Breast | MDA-A1 | 1,4-20,8 |
| | MDA-MB231 | 4,8-16,8 |
| Prostate | PC3 | 4,4-16,0 |
| Fibroblasts | NHDF | 6,2-9,5 |

| Table 2: In-vitro activity of compound (IX) | | |
|---|---|---|
| Cancer type | Cell line | IC₅₀ (nM) |
| Colon | HCT116 | 2,1-2,2 |
| Breast | MDA-A1 | 9,6-14,1 |
| | MDA-MB231 | 5,1-8,4 |

| Table 3: In-vitro activity of compounds (III), (V), (VI), (VII) and (VIII) | |
|---|---|
| Compound | Average IC₅₀ (nM) against HCT116 (colon) |
| III | 0,326 |
| V | 0,673 |
| VI | 0,393 |
| VII | 0,268 |
| VIII | 2,103 |

The invention therefore relates to the compound of the formula (I), wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached;
R1 and R2 independently of one another are H, Cl or Br;
R3 is H, (C₁-C₆)-alkyl, C(=O)-(C₁-C₆)-alkyl or (C₁-C₆)-alkylene-NH-(C₁-C₆)-alkyl;
R4 is H, (C₁-C₆)-alkyl or C(=O)-(C₁-C₆)-alkyl; and
R5 is methyl or ethyl;
or of a physiologically tolerated salt thereof, for use in the treatment and/or prophylaxis of cancer diseases, preferably for use in the treatment of breast cancer, intestinal cancer, stomach cancer, liver cancer, skin cancer, brain tumors, ovarial tumors, esophageal cancer, renal cancer and muscle cell carcinoma, in particular carcinoma of the head and neck muscles.

The present invention further relates to a pharmaceutical composition for use in the treatment and/or prophylaxis of cancer diseases, preferably for use in the treatment and/or prophylaxis of breast cancer, intestinal cancer, stomach cancer, liver cancer, skin cancer, brain tumors, ovarial tumors, esophageal cancer, renal cancer and muscle cell carcinoma, in particular carcinoma of the head and neck muscles, containing at least one compound of the formula (I), wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached;
R1 and R2 independently of one another are H, Cl or Br;
R3 is H, (C₁-C₆)-alkyl, C(=O)-(C₁-C₆)-alkyl or (C₁-C₆)-alkylene-NH-(C₁-C₆)-alkyl;
R4 is H, (C₁-C₆)-alkyl or C(=O)-(C₁-C₆)-alkyl; and
R5 is methyl or ethyl;
or a physiologically tolerable salt of a compound of the formula (I).

Additionally, the invention relates to a pharmaceutical composition containing at least one compound of the formula (I), wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached,
R1 and R2 independently of one another are H, Cl or Br,
R3 is H, (C₁-C₆)-alkyl, C(=O)-(C₁-C₆)-alkyl or (C₁-C₆)-alkylene-NH-(C₁-C₆)-alkyl,
R4 is H, (C₁-C₆)-alkyl or C(=O)-(C₁-C₆)-alkyl, and
R5 is methyl or ethyl,
provided that when
X and Y independently of one another are OH, O-(C₁-C₆)-alkyl, NH₂ or NH-(C₁-C₆)-alkyl, or X and Y together form a group -O-,
R1 and R2 independently of one another are H or Cl,
R3 is H, (C₁-C₆)-alkyl, C(=O)-(C₁-C₆)-alkyl or (C₁-C₆)-alkylene-NH-(C₁-C₆)-alkyl, and
R4 is H, (C₁-C₆)-alkyl or C(=O)-(C₁-C₆)-alkyl,
R5 may not be ethyl,
or a physiologically tolerable salt of a compound of the formula (I),
preferably for use in the treatment and/or prophylaxis of cancer diseases,
more preferred for use in the treatment and/or prophylaxis of breast cancer, intestinal cancer, stomach cancer, liver cancer, skin cancer, brain tumors, ovarial tumors, esophageal cancer, renal cancer and muscle cell carcinoma, in particular carcinoma of the head and neck muscles.

The compound or the compounds of the formula (I) can be administered as such in substance or preferably as a mixture with one or more of the customary pharmacologically suitable vehicles or excipients.

The compounds according to the invention are stable in the solid state and in solutions in the pH range between 2 and 9, in particular 5 and 7, and can thus be incorporated into customary galenical preparations.

The pharmaceutical composition according to the invention can be administered orally or parenterally, but rectal administration is also possible in principle. Suitable solid or liquid galenical preparation forms are, for example, granules, powders, tablets, coated tablets, (micro)capsules, suppositories, syrups, emulsions, suspensions, aerosols, drops or injectable solutions in ampoule form, and preparations with protracted release of active substance, in whose preparation pharmacologically suitable vehicles or excipients such as disintegrants, binders, coating agents, swelling agents, glidants or lubricants, flavor additives, sweeteners or solubilizers are customarily used, for example magnesium carbonate, titanium dioxide, lactose, mannitol and other sugars, talc, milk protein, gelatine, starch, vitamins, cellulose and its derivatives, animal or vegetable oils, polyethylene glycols and solvents, such as, for example, sterile water, alcohols, glycerol and polyhydric alcohols.

Optionally, the dose units for oral administration can be microencapsulated in order to delay release or to extend it over a longer period, such as, for example, by coating or embedding the active substance in particle form in suitable polymers, waxes.

Preferably, the pharmaceutical composition are prepared and administered in dose units, where each unit contains as active constituent a certain dose of one or more compounds of the natural substance derivatives according to the invention. In the case of solid dose units such as tablets, capsules and suppositories, this dose can be up to approximately 500 mg, but preferably approximately 0.1 to 200 mg, and in the case of injection solutions in ampoule form up to approximately 200 mg, but preferably approximately 0.5 to 100 mg, per day.

The daily dose to be administered is dependent on the body weight, age, sex and condition of the mammal. Under certain circumstances, however, higher or lower daily doses can also be appropriate. The administration of the daily dose can be carried out both by single administration in the form of an individual dose unit or else in a number of smaller dose units and by multiple administrations of subdivided doses at certain intervals.

The pharmaceutical compositions according to the invention are prepared by optionally mixing one or more of the compounds of the formula (I) with one or more of the customary vehicles or excipients, and bringing into a suitable administration form.

The following examples are intended to serve for the more detailed illustration of the invention, without restricting the breadth of the invention in any way.

Percentages relate to the weight. Mixing ratios in the case of liquids relate to the volume, if no other particulars have been given.

### Example 1: Storage of Nannocystis sp. ST 201196 (DSM 18870) at -135°C

An agar plate (0.5% fresh baker's yeast; 0.1% CaCl₂ x 2 H₂O; 0.477% HEPES (20 mM); 0.00005% cyanocobalamin; 1.8% agar; pH 7.2) was inoculated with the strain *Nannocystis sp.* ST 201196 (DSM 18870) and incubated for about 7-10 days at 30°C. The cells of this surface culture were scraped from the agar surface using a sterile spatula, suspended in 1 ml of Casitone medium (1% Casitone (Difco); 0.15% MgSO₄ x 7H₂O; 25% glycerine; pH 7.0) in cryotubes and stored at -135°C.

### Example 2: Storage of Nannocystis sp. ST 201196 (DSM 18870) at -196°C

An agar plate (0.5% fresh baker's yeast; 0.1 % CaCl₂ x 2H₂O; 0.477% HEPES (20 mM); 0.00005% cyanocobalamin; 1.8% agar; pH 7.2) was inoculated with the strain *Nannocystis sp.* ST 201196 (DSM 18870) and incubated for about 7-10 days at 30°C. The cells of this surface culture were scraped from the agar surface using a sterile spatula, suspended in 1 ml of Casitone medium (1% Casitone (Difco); 0.15% MgSO₄ x 7H₂O; 25% glycerine; pH 7.0) in cryotubes and stored at -196°C.

### Example 3: Preparation of a pre-culture of ST 201196 (DSM 18870) in an Erlenmeyer flask

100 ml of nutrient solution (1% fresh baker's yeast; 0.1 % CaCl₂ x 2H₂O; 0.477% HEPES (20 mM); 0.00005% cyanocobalamin; pH 7.2) in a sterile 300 ml Erlenmeyer flask were inoculated with the strain *Nannocystis sp.* ST 201196 (DSM 18870) and incubated for 6-8 days at 30°C and 180 rpm on a rotary shaker. 50 ml of this pre-culture were subsequently used for the preparation of each 500 ml main culture (10% inoculum).

### Example 4: Preparation of a liquid main culture of Nannocystis sp. ST 201196 (DSM 18870)

A sterile 2 l Erlenmeyer flask containing 500 ml of the following nutrient solution (0.5 % soybean meal; 0.1% glucose; 1% soluble starch; 0.1 % CaCl₂ x 2H₂O; 0.1 % MgSO₄ x 7H₂O; 0.477% HEPES (20 mM); 0.00005% cyanocobalamin; 2% of the adsorber resin XAD-16, pH 7,2) was inoculated with 50 ml (10%) of a pre-culture (see Example 3) or a culture grown on a fresh agar plate (0.5% fresh baker's yeast; 1 % CaCl₂ x 2 H₂O; 0.477% HEPES (20 mM); 0.00005% cyanocobalamin; 1.8% agar; pH 7.2) and incubated on a shaker at 180 rpm and 30°C. The maximum production of the substances according to the invention was reached after 144 to 216 hours.

### Example 5: Preparation of the substances (II), (III) and (V) in the fermenter

The 10 l and 50 l fermenters were operated under the following conditions:

| | |
|---|---|
| Inoculum: | 20% of pre-culture (see Example 3) |
| Nutrient medium: | 0.5% soybeanmeal; 0.1% glucose; 1% soluble starch; 0.1% CaCl₂ x 2H₂O; 0.1 % MgSO₄ x 7H₂O; 0.477% HEPES (20 mM); 0.00005% cyanocobalamin, 2% of the adsorber resin XAD-16 |
| Incubation temperature: | 30°C |
| Stirrer speed: | 200 rpm |
| Aeration: | 0.5 vvm |
| pH regulation: | none, before sterilization pH 7.6 ± 0.3 by means of KOH |
| pO₂ regulation: | none |
| Antifoam additive: | 0.05% Desmophen (Bayer) |
| Run time: | 144-216 h |

The pH regulation was carried out using 10% KOH, or 10% H₂SO₄.

### Example 6: Preparation of a liquid main culture of Nannocystis sp. ST 201196 (DSM 18870) with calcium bromide

A sterile 2 l Erlenmeyer flask containing 500 ml of the following nutrient solution (0.5 % soybeanmeal; 0.1% glucose; 1% soluble starch; 0.1% CaBr₂ · xH₂O; 0.1% MgSO₄ x 7H₂O; 0.477% HEPES (20 mM); 0.00005% cyanocobalamin; 2% of the adsorber resin XAD-16, pH 7,2) was inoculated with 50 ml (10%) of a pre-culture (see Example 3) or a culture grown on a fresh agar plate (0.5% fresh baker's yeast; 1 % CaCl₂ x 2 H₂O; 0.477% HEPES (20 mM); 0.00005% cyanocobalamin; 1.8% agar; pH 7.2) and incubated on a shaker at 180 rpm and 30°C. The maximum production of the substances according to the invention was reached after 144 to 216 hours.

### Example 7: Preparation of the substances (VI), (VII) and (VIII) in the fermenter

The 10 l and 50 l fermenters were operated under the following conditions:

| | |
|---|---|
| Inoculum: | 20% of pre-culture (see Example 3) |
| Nutrient medium: | 0.5% soybeanmeal; 0.1% glucose; 1% soluble starch; 0.1% CaBr₂ · xH₂O; 0.1% MgSO₄ x 7H₂O; 0.477% HEPES (20 mM); 0.00005% cyanocobalamin, 2% of the adsorber resin XAD-16 |
| Incubation temperature: | 30°C |
| Stirrer speed: | 200 rpm |
| Aeration: | 0.5 vvm |
| pH regulation: | none, before sterilization pH 7.6 ± 0.3 by means of KOH |
| pO₂ regulation: | none |
| Antifoam additive: | 0.05% Desmophen (Bayer) |
| Run time: | 144-216 h |

The pH regulation was carried out using 10% KOH, or 10% H₂SO₄.

### Example 8: Liquid-liquid extraction of compounds (II), (III) and (V)

After completion of the fermentation of the microorganism strain *Nannocystis sp.* ST 201196 (DSM 18870), the culture broth from example 4 (∼ 90 L of culture broth) was filtered. The biomass containing the XAD was lyophilized and subsequently extracted with methanol (5 x 10 L). The methanol extract was reduced to 1 L in vacuo. 1 L water was added and the water/methanol layer was then extracted four times with 1 L dichloromethane. Afterwards the dichloromethane layer was completely reduced to dryness to give a brown oil.

### Example 9: Pre-separation of the compounds (II), (III) and (V) by RP-18 chromatography

The complete dichloromethane extract from example 8 was successively purified in 500 mg steps. For example: 500 mg of the dichloromethane extract was dissolved in a mixture of methanol / dimethylsulfoxide / 2-propanol (3 ml, 20:70:10) and loaded onto a Phenomenex Luna® 10µ C18 (2) column (dimensions: 50 x 100 mm) having a Waters XTerra® Prep MS C18 10µ pre-column (dimension: 19 x 10 mm). Compounds were eluted with a gradient from 5% to 95% acetonitrile in water over a period of 27 min at a flow rate of 120 ml/min. The buffer (0.65 M ammonium acetate, pH 4.6 adjusted with acetic acid) was pumped into the system with a flow rate of 1.2 ml/min. Fractions were collected by UV (220 nm). Fraction 23 and 24 contained compound (V) and (II). They were combined and freeze dried. Fraction 22 was also lyophilized and contained compound (III).

### Example 10: Final purification of compounds (II) and (V) by RP-18 chromatography

Fraction 23 and 24 from example 9 were dissolved in a mixture of methanol / dimethylsulfoxide / 2-propanol (1 ml, 20:70:10) and loaded onto a Phenomenex Luna® 10µ C18 (2) column (dimensions: 50 x 100 mm) having a Waters XTerra® Prep MS C18 10µ pre-column (dimension: 19 x 10 mm). Compounds were eluted with a gradient from 5% to 40% acetonitrile in water within 5 min and from 40% to 95% within 27 min at a flow rate of 120 ml/min. The buffer (0.65 M ammonium acetate, pH 9 adjusted with aqueous ammonia) was pumped into the system with a flow rate of 1.2 ml/min. Fractions were collected by UV (220 nm). Fraction 32 and 33 were combined and afforded after lyophilization pure (II). Fraction 28 and 29 were also combined and afforded after freeze drying pure (V).

### Example 11: Final purification of compound (III) by RP-18 chromatography

Fraction 22 from example 9 was dissolved in a mixture of methanol /dimethylsulfoxide / 2-propanol (1 ml, 20:70:10) and loaded onto a Phenomenex Luna® 10µ C18 (2) column (dimensions: 30 x 100 mm) having a Waters XTerra® Prep MS C18 10µ pre-column (dimension: 19 x 10 mm). The Compound was eluted with a gradient from 5% to 40% acetonitrile in water within 5 min and from 40% to 95% within 27 min at a flow rate of 70 ml/min. The buffer (0.65 M ammonium acetate, pH 9 adjusted with aqueous ammonia) was pumped into the system with a flow rate of 0.7 ml/min. Fractions were collected by UV (220 nm). Fraction 13 was freeze dried and afforded compound (III).

### Example 12: Solid-phase extraction of compounds (II), (III) and (V)

After completion of the fermentation of the microorganism strain *Nannocystis sp.* ST 201196 (DSM 18870), the culture broth from Example 4 (∼ 80 L of culture broth) was filtered. The biomass containing the XAD was lyophilized and subsequently extracted with methanol (5 x 10 L). The methanol layer was concentrated in vacuo to a volume of 4 L and subsequently applied to a prepared column, which was filled with about 3.0 liters of CHP-20P material (MCI® Gel, 75-150µ, Mitsubishi Chemical Corporation). Elution was carried out using a methanol gradient from 10% to 95%. The column flow (120 ml/min) was collected in fractions (1 l fractions). The fractions containing compounds (III), (V) and (II) were combined, the solvent was removed on a Rotavapor and the fraction pool was subsequently lyophilized. That fraction could be further purified using the procedures described in examples 9-11

### Example 13: Liquid-liquid extraction of compounds (VI), (VII) and (VIII)

After completion of the fermentation of the microorganism strain *Nannocystis sp.* ST 201196 (DSM 18870), the culture broth from Example 6 (∼ 90 L of culture broth) was filtered. The biomass containing the XAD was lyophilized and subsequently extracted with methanol (5 x 10 L). The methanol extract was reduced to 1 L in vacuo. 1 L water was added and the water/methanol layer was then extracted four times with 1 L dichloromethane. Afterwards the dichloromethane layer was completely reduced to dryness to give a brown oil.

### Example 14: Pre-separation of the compounds (VI), (VII) and (VIII) by RP-18 chromatography

The complete dichloromethane extract from example 13 was successively purified in 5 g steps. For example: 5 g of the dichloromethane extract were dissolved in 50 ml methanol and loaded onto a Phenomenex Luna® 10µ C18 (2) column (dimensions: 50 x 250 mm) having a Waters XTerra® Prep MS C18 10µ pre-column (dimension: 19 x 10 mm). Compounds were eluted with a gradient from 5% to 95% acetonitrile in water over a period of 40 min at a flow rate of 150 ml/min. The buffer (0.65 M ammonium acetate, pH 4.6 adjusted with acetic acid) was pumped into the system with a flow rate of 1.5 ml/min. Fractions were collected by time (1 min per fraction). Fraction 29 contained compounds (VII) and (VI) and fraction 31 contained compound (VIII). Both fractions were freeze dried.

### Example 15: Pre-separation of compounds (VI) and (VII) by RP-18 chromatography

Fraction 29 from example 14 was dissolved in a mixture of methanol / dimethylsulfoxide / 2-propanol (1 ml, 20:70:10) and loaded onto a Phenomenex Luna® 10µ C18 (2) column (dimensions: 30 x 100 mm) having a Waters XTerra® Prep MS C18 10µ pre-column (dimension: 19 x 10 mm). Compounds were eluted with a gradient from 5% to 95% acetonitrile in water over a period of 27 min at a flow rate of 60 ml/min. The buffer (0.65 M ammonium acetate, pH 9 adjusted with aqueous ammonia) was pumped into the system with a flow rate of 0.6 ml/min. Fractions were collected by UV (220 nm). Fraction 74 was freeze dried and contained compound (VII) and (VI).

### Example 16: Pre-separation of compound (VIII) by RP-18 chromatography

Fraction 31 from example 14 was dissolved in a mixture of methanol / dimethylsulfoxide / 2-propanol (1 ml, 20:70:10) and loaded onto a Phenomenex Luna® 10µ C18 (2) column (dimensions: 50 x 100 mm) having a Waters XTerra® Prep MS C18 10µ pre-column (dimension: 19 x 10 mm). Compounds were eluted with a gradient from 5% to 95% acetonitrile in water over a period of 27 min at a flow rate of 120 ml/min. The buffer (0.65 M ammonium acetate, pH 9 adjusted with aqueous ammonia) was pumped into the system with a flow rate of 1.2 ml/min. Fractions were collected by UV (220 nm). Fractions 48-50 were combined and freeze dried and contained compound (VIII).

### Example 17: Final purification of compounds (VI) and (VII) by RP-18 chromatography

Fraction 74 from example 15 was dissolved in a mixture of methanol / dimethylsulfoxide / 2-propanol (1 ml, 20:70:10) and loaded onto a Phenomenex Luna® 10µ C18 (2) column (dimensions: 21 x 100 mm) having a Waters XTerra® Prep MS C18 10µ pre-column (dimension: 19 x 10 mm). Compounds were eluted with a gradient from 5% to 40% acetonitrile in water within 5 min and from 40% to 95% within 27 min at a flow rate of 45 ml/min. The buffer (10 % formic acid in water, pH 2) was pumped into the system with a flow rate of 0.5 ml/min. Fractions were collected by UV (220 nm). Fraction 24 was freeze dried and gave pure compound (VII) and fraction 27 was freeze dried to give pure compound (VI).

### Example 18: Final purification of compound (VIII) by RP-18 chromatography

Fractions 48-50 from example 16 were dissolved in a mixture of methanol / dimethylsulfoxide / 2-propanol (1 ml, 20:70:10) and loaded onto a Phenomenex Luna® 10µ C18 (2) column (dimensions: 21 x 100 mm) having a Waters XTerra® Prep MS C18 10µ pre-column (dimension: 19 x 10 mm). Compounds were eluted with a gradient from 5% to 95% acetonitrile in water within 27 min at a flow rate of 45 ml/min. The buffer (10 % formic acid in water, pH 2) was pumped into the system with a flow rate of 0.5 ml/min. Fractions were collected by UV (220 nm). Fraction 17 was freeze dried to give pure compound (VIII).

### Example 19: Solid-Phase extraction of compounds (VI), (VII) and (VIII)

After completion of the fermentation of the microorganism strain *Nannocystis sp.* ST201196 (DSM 18870), the culture broth from Example 6 (∼ 90 L of culture broth) was filtered. The biomass containing the XAD was lyophilized and subsequently extracted with methanol (5 x 10 L). The methanol layer was concentrated in vacuo to a volume of 4 L and subsequently applied to a prepared column, which was filled with about 3.0 liters of CHP-20P material (MCI® Gel, 75-150µ, Mitsubishi Chemical Corporation). Elution was carried out using a methanol gradient of from 10% to 95%. The column flow (120 ml/min) was collected in 1 l fractions. Fractions containing compounds (VII), (VI) and (VIII) were combined, the solvent was removed on a Rotavapor and the fraction pool was subsequently lyophilized. That fraction could be further purified following the procedures described in examples 14 to 18.

### Example 20: Characterization of compounds (II), (III), (IV), (V), (VI), (VII), (VIII) and (X) by high performance liquid chromatography with diode-array and mass spectrometry detection (HPLC-DAD-MS)

The macrolactons (II), (III), (IV), (V), (VI), (VII), (VIII) and (X) were analyzed on a Waters Acquity UPLC System with Sample Manager, Binary Solvent Manager and PDA (Detector). As UPLC column a Waters Acquity UPLC BEH C18 (1.7µ; 2.1 x 100 mm) was used and eluted at a flow rate of 0.6 ml/min with a gradient of water:acetonitrile (9:1) within 15 min to 100% acetonitrile, all solvents buffered with 6.5 mM ammoniumacetate to pH 4.6. UV spectra were recorded by the PDA detector at wavelengths between 200 and 500 nm. Mass-Spectra were recorded with a Bruker µTOF LC-MS using an orthogonal electrospray ionisation, a sampling-rate of 0.5 Hz and a detection-limit of 150-1500 mu.

### Example 21: Characterization of compound (II)

Macrolacton (II) eluted at 9.22 min (TIC). The UV spectrum is featured by λₘₐₓ of 204, 232 and 286 nm.

Molecular ions were observed at m/z (I): 814.3, M-H (-), (100); 815.3 (47); 816.3 (73); 817.3 (30); 818.3 (16) in negative mode. Molecular ions were observed at m/z (I): 816.3, M+H (+), (17); 817.3 (10); 818.3 (14); 819.3 (6); 833.3, M+NH4 (+), (100); 834.3 (44); 835.3 (74); 836.3 (29); 837.3 (16) in positive mode.

| | |
|---|---|
| Exact, mono-isotopic mass of neutral [M] | 815.3310 |
| Theoretical mass [M] for C₄₂H₅₅Cl₂N₃O₉ | 815.3315 |
| Molecular formula | C₄₂H₅₅Cl₂N₃O₉ |

### Example 22: Characterization of compound (III)

Macrolacton (III) eluted at 8.95 min (TIC). The UV spectrum is featured by λₘₐₓ of 207 and 232 nm.

Molecular ions were observed at m/z (I): 780.4, M-H (-), (100); 781.4 (48.1); 782.4 (44.6); 783.4 (10.5) in negative mode. Molecular ions were observed at m/z (I): 782.4, M+H (+), (25); 783.4 (15); 784.4 (11); 799.4, M+NH4 (+), (100); 800.4 (47.4); 801.4 (43.6); 802.4 (16); 803.4 (3.3) in positive mode.

| | |
|---|---|
| Exact, mono-isotopic mass of neutral [M] | 781.3710 |
| Theoretical mass [M] for C₄₂H₅₆ClN₃O₉ | 781.3705 |
| Molecular formula | C₄₂H₅₆ClN₃O₉ |

NMR-chemical shifts of compound (III) in d₆-DMSO at 300 K; c = 3 mg/ml.

| position | δ (¹³C) [ppm] | δ (¹H) [ppm] |
|---|---|---|
| 1 | 126,95 | 7.259 |
| 2 | 127,79 | 7.291 |
| 3 | 125,98 | 7.568 |
| 4 | 140,00 | |
| 5 | 78,84 | 5.909 |
| 6 | 41,92 | 2.639 |
| 6-Me | 9,56 | 0.963 |
| 7 | 138,44 | 6.174 |
| 8 | 124,65 | 6.398 |
| 9 | 128,94 | 6.1222 |
| 10 | 133,35 | |
| 10-Me | 10,69 | 1.700 |
| 11 | 83,98 | 3.643 |
| 11-Me | 55,15 | 3.101 |
| | | 2.110 |
| 12 | 30,57 | 1.473 |
| 13 | 58,08 | 2.656 |
| 14 | 61,35 | |
| 14-Me | 14,86 | 1.446 |
| 15 | 169,32 | |
| 16 N-Me | 29,70 | 2.978 |
| 17a | 59,20 | 4.465 |
| b | 31,10 | 1.703 |
| b-Me | 14,55 | 0.325 |
| | | 1.210 |
| c | 24,25 | 0.861 |
| d | 10,06 | 0.760 |
| 18 CO | 168,69 | |
| 19 NH | | 8.001 |

### Example 23: Characterization of compound (IV)

Macrolacton (IV) eluted at 8.28 min (TIC). The UV spectrum is featured by λₘₐₓ of 231 nm.

Molecular ions were observed at m/z (I): 746.4, M-H (-), (100); 747.4 (37.6) in negative mode. Molecular ions were observed at m/z (I): 748.4, M+H (+), (35.2); 749.4 (14.9); 750.4 (3.4); 765.4, M+NH4 (+), (100); 766.5 (42.3); 767.5 (9.3) in positive mode.

| | |
|---|---|
| Exact, mono-isotopic mass of neutral [M] | 747.4083 |
| Theoretical mass [M] for C₄₂H₅₇N₃O₉ | 747.4095 |
| Molecular formula | C₄₂H₅₇N₃O₉ |

### Example 24: Characterization of compound (V)

Macrolacton (V) eluted at 9.00 min (TIC). The UV spectrum is featured by λₘₐₓ of 204, 230 and 286 nm.

Molecular ions were observed at m/z (I): 800.3, M-H (-), (100); 801.3 (40); 802.3 (70.2); 803.3 (24), 804.3 (11.5) in negative mode. Molecular ions were observed at m/z (I): 802.3, M+H (+), (18); 804.3 (12); 819.3494, M+NH4 (+), (100); 820.3518 (42.9); 821.3479 (74.5); 822.3 (23.7); 823 (14) in positive mode.

| | |
|---|---|
| Exact, mono-isotopic mass of neutral [M] | 801.3156 |
| Theoretical mass [M] for C₄₁H₅₃Cl₂N₃O₉ | 801.3159 |
| Molecular formula | C₄₁H₅₃Cl₂N₃O₉ |

NMR-chemical shifts of compound (V) in d₆-DMSO at 300 K; c = 3 mg/ml.

| position | | δ (¹³C) | δ (¹H) |
|---|---|---|---|
| 1 | | 126.89 | 7.251 |
| 2 | (2C) | 127.73 | 7.322 |
| 3 | (2C) | 125.91 | 7.559 |
| 4 | | 139.90 | |
| 5 | | 78.79 | 5.891 |
| 6 | | 41.85 | 2.652 |
| 6-Me | | 9.61 | 0.961 |
| 7 | | 138.22 | 6.178 |
| 8 | | 124.66 | 6.382 |
| 9 | | 128.74 | 6.115 |
| 10 | | 133.30 | |
| 10-Me | | 10.73 | 1.698 |
| 11 | | 83.84 | 3.620 |
| 11-OMe | | 55.10 | 3.092 |
| 12 | | | 1.472 |
| | | 30.50 | 2.119 |
| 13 | | 57.97 | 2.628 |
| 14 | | 61.21 | |
| 14-Me | | 14.85 | 1.434 |
| 15 | | 169.39 | |
| 16 N-Me | | 29.46 | 2.980 |
| 17 α | | 61.01 | 4.388 |
| β | | 25.28 | 1.867 |
| γ | | 18.53 | 0.700 |
| γ' | | 18.44 | 0.388 |
| 18 | CO | 168.52 | |
| 19 | NH | 123.6 | 8.059 |
| 20 | α | 52.64 | 4.678 |
| | β | | 2.498 |
| | | 37.04 | 2.809 |
| | γ | 130.69 | |
| | δ | 129.62 | 7.360 |
| | ε | 121.47 | |
| | ϕ | 147.31 | |
| | ϕ-OH | | 9.77 br |
| 21 | CO | 170.40 | |
| 22 | NH | 114.0 | 8.616 |
| 23 | α | 59.18 | 4.679 |
| | β | 71.80 | |
| | β-OH | | 5.15 |
| | γ | 24.41 | 1.034 |
| | γ' | 28.14 | 1.139 |
| 24 | CO | 170.57 | |

### Example 25: Characterization of compound (VI)

Macrolacton (VI) eluted at 9.50 min (TIC). The UV spectrum is featured by λₘₐₓ of 207 and 232 nm.

Molecular ions were observed at m/z (I): 858.3, M-H(-), (67.7); 859.3 (30.5); 860.3 (100); 861.3 (40.9); 862.3 (30.2); 863.3 (10.4); in the negative mode. Molecular ions were observed at m/z (I): 877.3, M+NH4 (+), (70); 878.3 (31.1); 879.3 (100); 880.3 (42.4); 881.3 (30.8); 882.3 (11) in positive mode.

| | |
|---|---|
| Exact, mono-isotopic mass of neutral [M] | 859.2800 |
| Theoretical mass [M] for C₄₂H₅₅BrClN₃O₉ | 859.2810 |
| Molecular formula | C₄₂H₅₅BrClN₃O₉ |

NMR-chemical shifts of compound (VI) in d₆-DMSO at 300 K; c = 3 mg/ml.

| position | | δ (¹³C) | δ (¹H) |
|---|---|---|---|
| 1 | | 126.93 | 7.249 |
| 2 | (2C) | 127.78 | 7.322 |
| 3 | (2C) | 125.94 | 7.562 |
| 4 | | 139.98 | |
| 5 | | 78.85 | 5.895 |
| 6 | | 41.90 | 2.655 |
| 6-Me | | 9.48 | 0.960 |
| 7 | | 138.45 | 6.184 |
| 8 | | 124.59 | 6.380 |
| 9 | | 128.97 | 6.120 |
| 10 | | 133.32 | |
| 10-Me | | 10.63 | 1.697 |
| 11 | | 84.02 | 3.617 |
| 11-OMe | | 55.13 | 3.090 |
| 12 | | 30.53 | 2.124 |
| | | | 1.460 |
| 13 | | 58.06 | 2.629 |
| 14 | | 61.37 | |
| 14-Me | | 14.87 | 1.430 |
| 15 | | 169.27 | |
| 16 N-Me | | 29.67 | 2.980 |
| 17 | α | 59.14 | 4.477 |
| | β | 31.01 | 1.697 |
| | β-Me | 14.55 | 0.348 |
| γ | | 24.23 | 0.871 |
| | | | 1.212 |
| δ | | 10.06 | 0.774 |
| 18 CO | | 168.71 | - |
| 19 NH | | 123.7 | 8.063 |
| 20 | α | 52.75 | 4.691 |
| | β | 36.92 | 2.496 |
| | | | 2.813 |
| | γ | 131.3 br | |
| | δ | 130.30 | 7.400 |
| | ε | 121.20 | |
| | ϕ | 148.27 | - |
| | ϕ-OH | | 9.87 |
| | ε' | 111.50 | - |
| | δ' | 132.64 | 7.505 |
| 21 | CO | 170.64 | - |
| 22 | NH | 114.0 | 8.620 |
| 23 | α | 59.14 | 4.687 |
| | β | 71.93 | |
| | β-OH | | 5.160 |
| | γ | 28.21 | 1.145 |
| | γ' | 24.35 | 1.033 |
| 24 | CO | 170.48 | |

### Example 26: Characterization of compound (VII)

Macrolacton (VII) eluted at 9.05 min (TIC). The UV spectrum is featured by λₘₐₓ of 232 nm.

Molecular ions were observed at m/z (I): 824.3, M-H (-), (89.5); 825.3 (39.2); 826.3 (100); 827.3 (39.7); 828.3 (9.6) in negative mode. Molecular ions were observed at m/z (I): 826.3, M+H (+), (89.9); 827.3 (39.5); 828.3 (100); 829.3 (39.8); 830.3 (9.4) in positive mode.

| | |
|---|---|
| Exact, mono-isotopic mass of neutral [M] | 825.3234 |
| Theoretical mass [M] for C₄₂H₅₆BrN₃O₉ | 825.3200 |
| Molecular formula | C₄₂H₅₆BrN₃O₉ |

NMR-chemical shifts of compound (VII) in d₆-DMSO at 300 K; c = 3 mg/ml.

| position | | δ (¹³C) | δ (¹H) |
|---|---|---|---|
| 1 | | 126.88 | 7.255 |
| 2 | (2C) | 127.71 | 7.328 |
| 3 | (2C) | 125.93 | 7.563 |
| 4 | | 139.91 | |
| 5 | | 78.77 | 5.904 |
| 6 | | 41.84 | 2.652 |
| 6-Me | | 9.61 | 0.967 |
| 7 | | 138.25 | 6.175 |
| 8 | | 124.65 | 6.382 |
| 9 | | 128.78 | 6.123 |
| 10 | | 133.30 | |
| 10-Me | | 10.69 | 1.700 |
| 11 | | 83.86 | 3.627 |
| 11-OMe | | 55.10 | 3.096 |
| 12 | | 30.53 | 1.469 |
| | | | 2.115 |
| 13 | | 57.99 | 2.641 |
| 14 | | 61.23 | |
| 14-Me | | 14.82 | 1.430 |
| 15 | | 169.28 | |
| 16 N-Me | | 29.63 | 2.980 |
| 17 | α | 59.08 | 4.469 |
| | β | 31.05 | 1.701 |
| | β-Me | 14.59 | 0.318 |
| | γ | 24.17 | 0.860 |
| | | | 1.217 |
| | δ | 10.01 | 0.769 |
| 18 | CO | 168.59 | - |
| 19 | NH | | 8.004 |
| 20 | α | 52.70 | 4.710 |
| | β | 37.18 | 2.492 |
| | | | 2.821 |
| | γ | n.d. | |
| | δ | 133.60 | 7.490 |
| | ε | 108.61 | - |
| | ϕ | 152.25 | - |
| | ϕ-OH | | 9.87 |
| | ε' | 115.49 | 6.745 |
| | δ' | 129.85 | 7.129 |
| 21 | CO | 170.70 | - |
| 22 | NH | | 8.633 |
| 23 | α | 59.20 | 4.683 |
| | β | 71.85 | |
| | β-OH | | 5.124 |
| | γ | 28.16 | 1.145 |
| | γ' | 24.41 | 1.032 |
| 24 | CO | 170.54 | |

### Example 27: Characterization of compound (VIII)

Macrolacton (VIII) eluted at 9.55 min (TIC). The UV spectrum is featured by λₘₐₓ of 230 and 284 nm.

Molecular ions were observed at m/z (I): 808.3, M-H (-), (87.2); 809.3 (33); 810.3 (100); 811.3 (32) in negative mode. Molecular ions were observed at m/z (I): 810.3, M+H (+), (89.6); 811.3 (34.5); 812.3 (100); 813.3 (33) in positive mode.

| | |
|---|---|
| Exact, mono-isotopic mass of neutral [M] | 809.3241 |
| Theoretical mass [M] for C₄₂H₅₆BrN₃O₈ | 809.3251 |
| Molecular formula | C₄₂H₅₆BrN₃O₈ |

NMR-chemical shifts of compound (VIII) in d₆-DMSO at 300 K; c = 3 mg/ml.

| position | | δ (¹³C) | δ (¹H) |
|---|---|---|---|
| 1 | | 126.96 | 7.253 |
| 2 | (2C) | 127.79 | 7.322 |
| 3 | (2C) | 125.96 | 7.534 |
| 4 | | 139.73 | - |
| 5 | | 78.77 | 5.867 |
| 6 | | 41.69 | 2.643 |
| 6-Me | | 10.19 | 0.933 |
| 7 | | 137.11 | 5.947 |
| 8 | | 125.03 | 6.348 |
| 9 | | 128.19 | 6.025 |
| 10 | | 133.46 | - |
| 10-Me | | 11.24 | 1.663 |
| 11 | | 84.77 | 3.563 |
| 11-OMe | | 55.02 | 3.092 |
| 12 | | | 2.371 |
| | | 30.52 | 2.361 |
| 13 | | 125.06 | 5.191 |
| 14 | | n.d | - |
| 14-Me | | 14.34 | 1.721 |
| 15 | | n.d | - |
| 16 N-Me | | 31.75 | 2.711 |
| 17 | α | 59.89 | 4.510 |
| | β | 31.02 | 1.777 |
| | β-Me | 15.04 | 0.467 d |
| | γ | | 1.224 |
| | | 23.96 | 0.906 |
| | δ | 10.18 | 0.769 t |
| 18 | CO | n.d | - |
| 19 | NH | | 7.708 |
| 20 | α | 53.04 | 4.723 |
| | β | | 2.700 |
| | | 36.62 | 2.374 |
| | γ | | - |
| | δ | 133.42 | 7.473 |
| | ε | 108.8 | - |
| | ϕ | 152.39 | - |
| | ϕ-OH | | |
| | ε' | 115.64 | 6.777 |
| | δ' | 129.73 | 7.117 |
| 21 | CO | | - |
| 22 | NH | | 8.456 |
| 23 | α | 59.34 | 4.660 |
| | β | 71.61 | - |
| | β-OH | | n.d., broad |
| | γ | 24.56 | 1.011 |
| | γ' | 28.71 | 1.159 |
| 24 | CO | | - |

### Example 28: Characterization of compound (X)

Macrolacton (X) eluted at 9.43 min (TIC). The UV spectrum is featured by λₘₐₓ of 231 and 279 nm.

Molecular ions were observed at m/z (I): 902.2, M-H(-), (45.6); 903.2 (25.0); 904.2 (100); 905.2 (45.4); 906.2 (54.0); 907.2 (22.4) in the negative mode. Molecular ions were observed at m/z (I): 921.3, M+NH4 (+), (49.0); 922.3 (20.6); 923.3 (100); 924.3 (40.1); 925.3 (71.3); 926.3 (30.4); 927.3 (9.1) in positive mode.

| | |
|---|---|
| Exact, mono-isotopic mass of neutral [M] | 903.2304 |
| Theoretical mass [M] for C₄₂H₅₅Br₂N₃O₉ | 903.2305 |
| Molecular formula | C₄₂H₅₅Br₂N₃O₉ |

### Example 29: In-vitro evaluation of the cell-proliferation for compounds (II)-(IX)

The anti-tumor activity of the compounds of the formula (I) was determined by measuring the inhibition of the cell proliferation of various tumor cell lines by the methods described below.

Experimental conditions for 14C-Thymidine incorporation assays:
1) Cells were seeded in 96 well Cytostar microplates in 180µl of culture medium (DMEM supplemented with 10% Fetal Calf Serum).
   Seeding density are 2500 cells/well for HCT116, 5000 cells/well for HT29 and PC3 and 10000 cells/well for MDA-MB231, MDA-A1, H460 and B16F10.
2) The microplates were incubated 4h at 37°C with 5% CO2
3) 10µl of test compound were added
4) The microplates were incubated 72h at 37°C with 5% CO2
5) 10µl of 14C-Thymidine at 10µCi/ml was added
6) The microplates were incubated 24h at 37°C with 5% CO2
7) The radioactivity incorporated in the cells was measured with a Microbeta Trilux radioactivity scintillation counter (Wallac).

Experimental conditions for ATP viability assays:
1) Cells were seeded in 96 well microplates in 135µl of culture medium.
   Culture medium used were RPMI supplemented with 10% Fetal Calf Serum for HL60 and CEM cell lines and DMEM with 10% FCS for Normal Human Dermal Fibroblasts (NHDF). Seeding density was 25000 cells/well for HL60 and CEM and 7500 cells/well for NHDF.
2) The microplates were incubated 4h at 37°C with 5% CO2.
3) 15µl of test compound were added
4) The microplates were incubated 96h.
5) 100µl of a mix of lysing reagent and luciferase/luciferin was added (CellTiterGlo, Promega). The luminescence emitted was measured with a Microbeta Trilux counter (Wallac).

| Cell lines | Tissue | Source | Reference |
|---|---|---|---|
| HL60 | Leukaemia | ATCC | CCL-240 |
| CEM | Leukaemia | ATCC | CCL-119 |
| NHDF | Fibroblast | Cambrex | CC-2511 |
| HCT116 | Colon | ATCC | CCL-247 |
| HT29 | Colon | ATCC | HTB-38 |
| H460 | NSCLC | ATCC | HTB-177 |
| B16F10 | Melanoma | NCl | Cancer Res. 1975, 35, 218-224 |
| MDA-A1 | Breast | INSERM | Cytometry 1991, 12(1), 15-25 |
| MDA-MB231 | Breast | ECACC | 92020424 |
| PC3 | Prostate | ATCC | CRL-1435 |

The results of the cell proliferation tests are shown in Tables 1, 2 and 3.

## Claims

1. Compound of the formula (I), 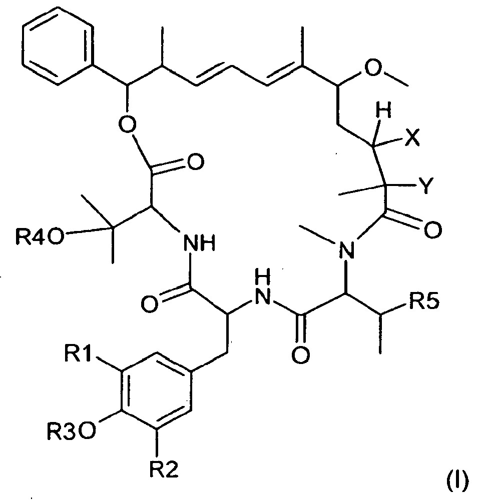 wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached,
R1 and R2 independently of one another are H, Cl or Br,
R3 is H, (C₁-C₆)-alkyl, C(=O)-(C₁-C₆)-alkyl or (C₁-C₆)-alkylene-NH-(C₁-C₆)-alkyl,
R4 is H, (C₁-C₆)-alkyl or C(=O)-(C₁-C₆)-alkyl, and
R5 is methyl or ethyl,
or a physiologically tolerable salt of a compound of the formula (I),
for use in the treatment and/or prophylaxis of cancer diseases.

2. Compound of the formula (I) for use according to claim 1, wherein X and Y together form a group -O- or wherein X and Y together form a further bond between the C atoms to which they are attached.

3. Compound of the formula (I) for use according to claim 1 or 2, wherein at least one of R1 and R2 is Cl or Br.

4. Compound of the formula (I) for use according to claims 1 to 3, wherein R3 is H or (C₁-C₆)-alkyl.

5. Compound of the formula (I) for use according to claims 1 to 4, wherein R4 is H.

6. Compound of the formula (I) for use according to claims 1 to 4, wherein R5 is ethyl.

7. Compound of the formula (I) for use according to claim 1, wherein
X and Y together form a group -O-, or X and Y form a further double bond between the C atoms to which they are attached,
R1 and R2 independently of one another are H, Cl or Br,
R3 and R4 independently of one another are H, (C₁-C₆)-alkyl or C(=O)-(C₁-C₆)-alkyl, and
R5 is methyl or ethyl.

8. Compound of the formula (I) for use according to claim 1, wherein
X and Y together form a group -O-, or X and Y form a further double bond between the C atoms to which they are attached,
R1 and R2 independently of one another are H, Cl or Br,
R3 is H or (C₁-C₆)-alkyl,
R4 is H, and
R5 is methyl or ethyl.

9. Compound of the formula (I) for use according to claim 1, wherein
X and Y together form a group -O-, or X and Y form a further double bond between the C atoms to which they are attached,
R1 and R2 are both Cl,
R3 is H or (C₁-C₆)-alkyl,
R4 is H, and
R5 is methyl or ethyl.

10. Compound of the formula (I) for use according to claim 1, wherein the compound of the formula (I) is a compound of the formulae (II) to (X).

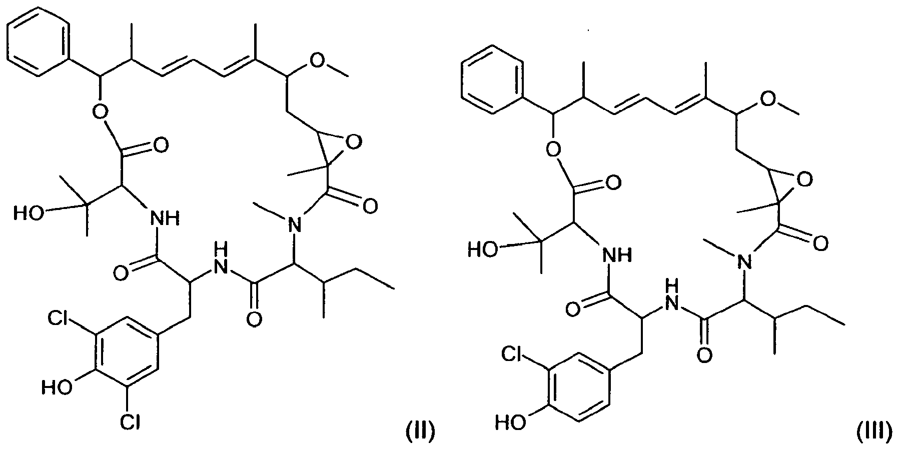

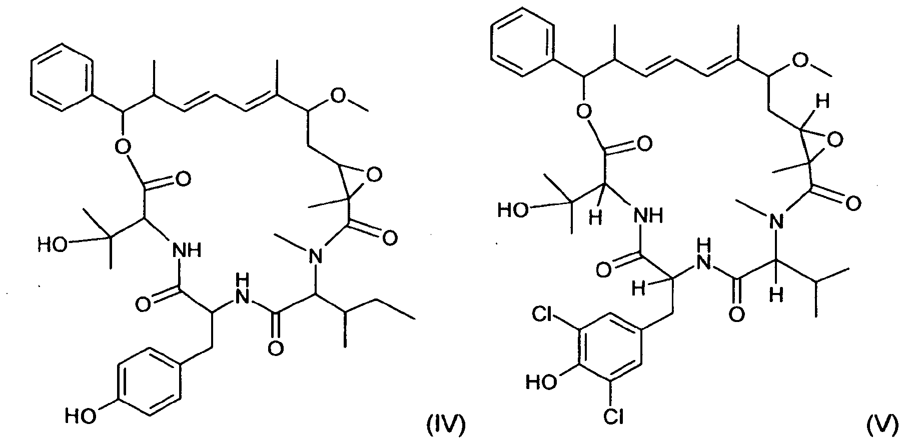

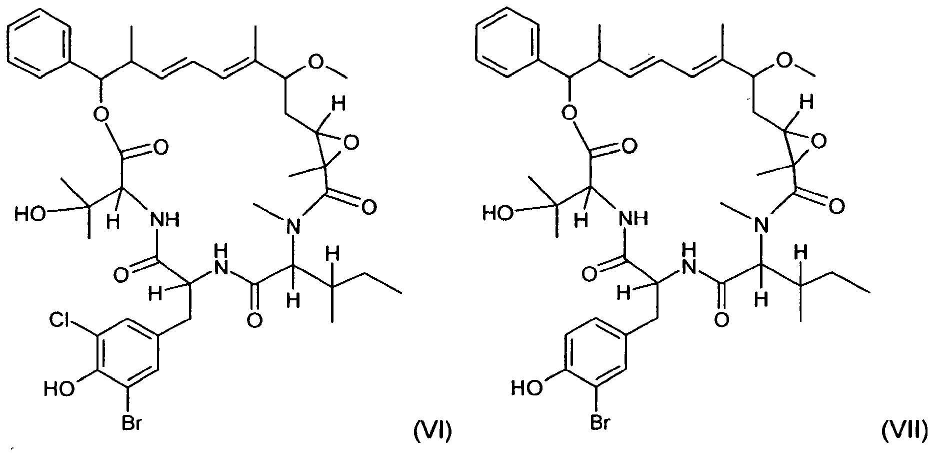

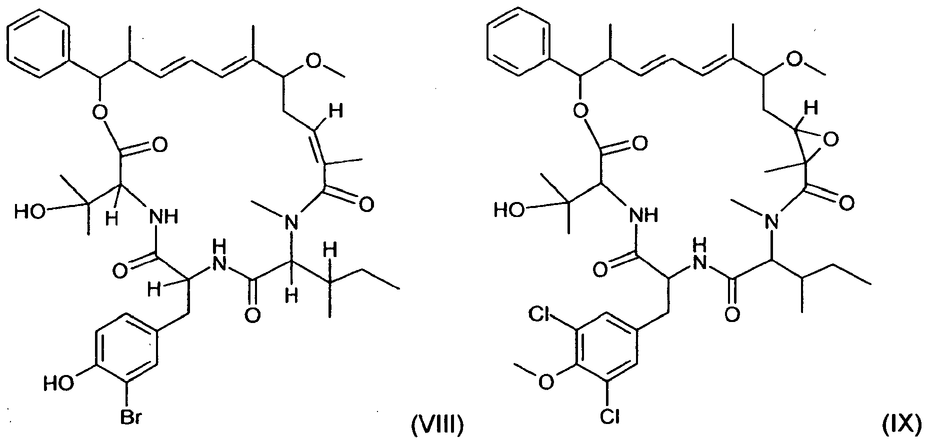

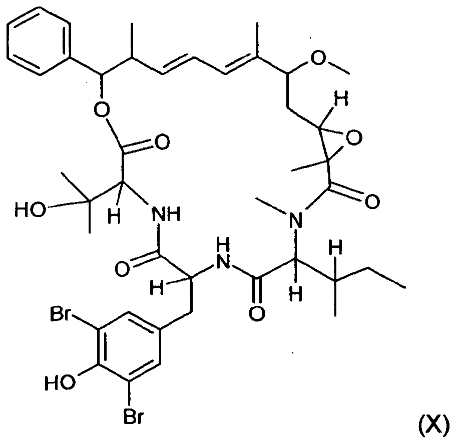

11. Pharmaceutical composition for use in the treatment and/or prophylaxis of cancer diseases, containing at least one compound of the formula (I) according to claims 1 to 10.

12. Compound of the formula (I), 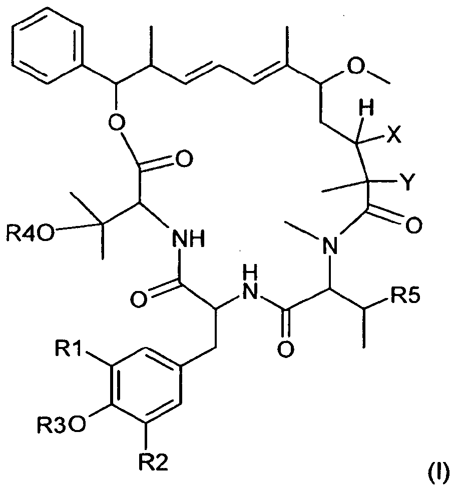 wherein
X and Y independently of one another are OH, O-(C₁-C₆)-alkyl, NH₂ or NH-(C₁-C₆)-alkyl, or X and Y together form a group -O- or wherein X and Y together form a further bond between the C atoms to which they are attached,
R1 and R2 independently of one another are H, Cl or Br,
R3 is H, (C₁-C₆)-alkyl, C(=O)-(C₁-C₆)-alkyl or (C₁-C₆)-alkylene-NH-(C₁-C₆)-alkyl,
R4 is H, (C₁-C₆)-alkyl or C(=O)-(C₁-C₆)-alkyl, and
R5 is methyl or ethyl,
provided that when
X and Y independently of one another are OH, O-(C₁-C₆)-alkyl, NH₂ or
NH-(C₁-C₆)-alkyl, or X and Y together form a group -O-,
R1 and R2 independently of one another are H or Cl,
R3 is H, (C₁-C₆)-alkyl, C(=O)-(C₁-C₆)-alkyl or (C₁-C₆)-alkylene-NH-(C₁-C₆)-alkyl, and
R4 is H, (C₁-C₆)-alkyl or C(=O)-(C₁-C₆)-alkyl,
R5 may not be ethyl,
or a physiologically tolerable salt of a compound of the formula (I).

13. Compound of the formula (I) according to claim 12, wherein the compound of the formula (I) is a compound of the formulae (V) to (VIII).

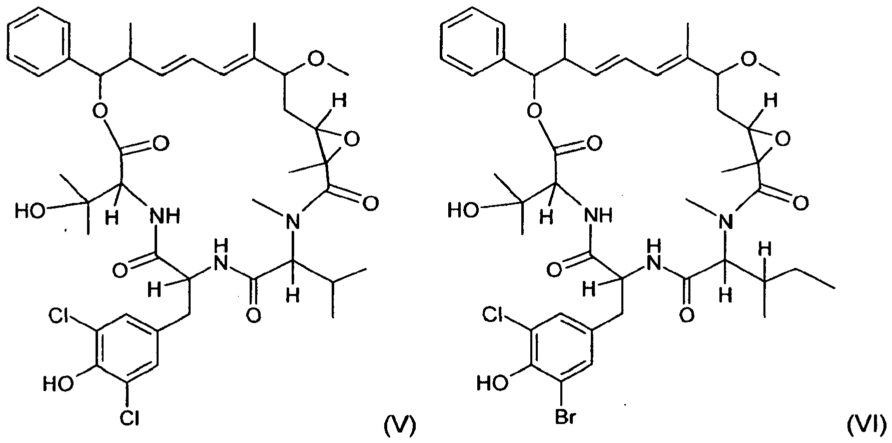

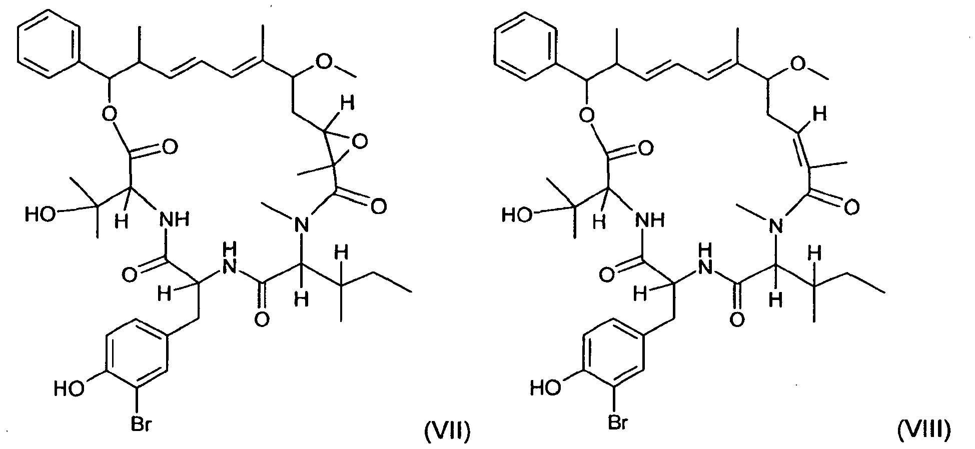

14. Process for the preparation of a compound of the formula (I) according to claim 12 or 13, or a physiologically tolerable salt thereof, which comprises
1. fermenting the strain *Nannocystis sp.* ST 201196 (DSM 18870) or one of its variants and/or mutants under suitable conditions in a culture medium which contains a Cl and/or a Br source, until one or more of the compounds of the formula (I) accumulates in the culture medium and
2. isolating a compound of the formula (I) from the culture medium, and
3. optionally derivatizing the compound of the formula (I) and/or converting it into a physiologically tolerable salt.

15. The process according to claim 14, wherein at least one Br source is present in the culture medium.

16. Pharmaceutical composition, containing at least one compound of the formula (I) according to claim 12 or 13.

## Patentansprüche

1. Verbindung der Formel (I), 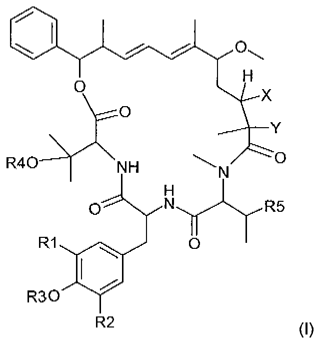 worin
X und Y unabhängig voneinander OH, O-(C₁-C₆)-Alkyl, NH₂ oder NH-(C₁-C₆)-Alkyl bedeuten, oder X und Y zusammen eine Gruppe -O- bilden oder wobei X und Y zusammen eine weitere Bindung zwischen den C-Atomen, an die sie gebunden sind, bilden,
R1 und R2 unabhängig voneinander H, Cl oder Br bedeuten,
R3 H, (C₁-C₆)-Alkyl, C(=O)-(C₁-C₆)-Alkyl oder (C₁-C₆)-Alkylen-NH-(C₁-C₆)-alkyl bedeutet,
R4 H, (C₁-C₆)-Alkyl oder C(=O)-(C₁-C₆)-Alkyl bedeutet, und
R5 Methyl oder Ethyl bedeutet,
oder ein physiologisch verträgliches Salz einer Verbindung der Formel (I), zur Verwendung bei der Behandlung und/oder Prophylaxe von Krebserkrankungen.

2. Verbindung der Formel (I) zur Verwendung nach Anspruch 1, wobei X und Y zusammen eine Gruppe -O- bilden oder wobei X und Y zusammen eine weitere Bindung zwischen den C-Atomen, an die sie gebunden sind, bilden.

3. Verbindung der Formel (I) zur Verwendung nach Anspruch 1 oder 2, wobei mindestens einer der Reste R1 und R2 Cl oder Br bedeutet.

4. Verbindung der Formel (I) zur Verwendung nach den Ansprüchen 1 bis 3, wobei R3 H oder(C₁-C₆)-Alkyl bedeutet.

5. Verbindung der Formel (I) zur Verwendung nach den Ansprüchen 1 bis 4, wobei R4 H bedeutet.

6. Verbindung der Formel (I) zur Verwendung nach den Ansprüchen 1 bis 4, wobei R5 Ethyl bedeutet.

7. Verbindung der Formel (I) zur Verwendung nach Anspruch 1, wobei X und Y zusammen eine Gruppe -O- bilden, oder X und Y eine weitere Doppelbindung zwischen den C-Atomen, an die sie gebunden sind, bilden,
R1 und R2 unabhängig voneinander H, Cl oder Br bedeuten,
R3 und R4 unabhängig voneinander H, (C₁-C₆)-Alkyl oder C(=O)-(C₁-C₆)-Alkyl bedeuten, und
R5 Methyl oder Ethyl bedeutet.

8. Verbindung der Formel (I) zur Verwendung nach Anspruch 1, wobei X und Y zusammen eine Gruppe -O- bilden, oder X und Y eine weitere Doppelbindung zwischen den C-Atomen, an die sie gebunden sind, bilden,
R1 und R2 unabhängig voneinander H, Cl oder Br bedeuten,
R3 H oder (C₁-C₆)-Alkyl bedeutet,
R4 H bedeutet, und
R5 Methyl oder Ethyl bedeutet.

9. Verbindung der Formel (I) zur Verwendung nach Anspruch 1, wobei X und Y zusammen eine Gruppe -O- bilden, oder X und Y eine weitere Doppelbindung zwischen den C-Atomen, an die sie gebunden sind, bilden,
R1 und R2 beide Cl bedeuten,
R3 H oder (C₁-C₆)-Alkyl bedeutet,
R4 H bedeutet, und
R5 Methyl oder Ethyl bedeutet.

10. Verbindung der Formel (I) zur Verwendung nach Anspruch 1, wobei es sich bei der Verbindung der Formel (I) um eine Verbindung der Formeln (II) bis (X) handelt.

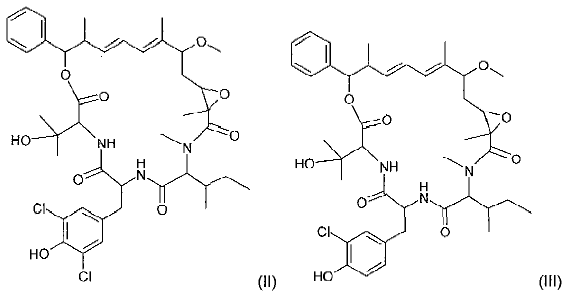

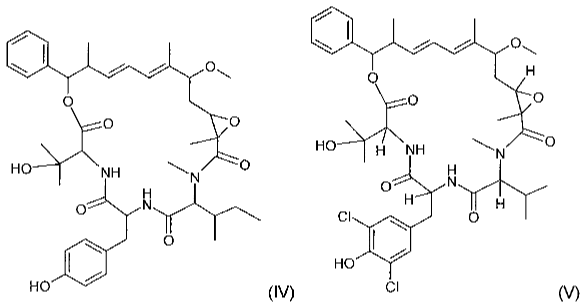

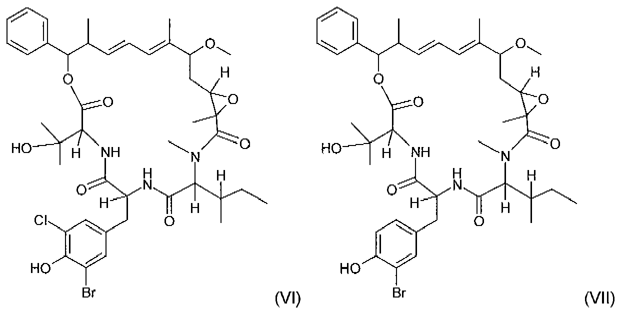

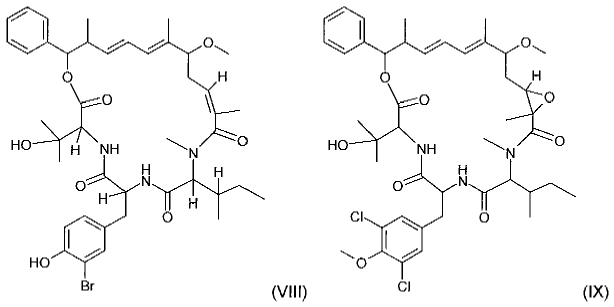

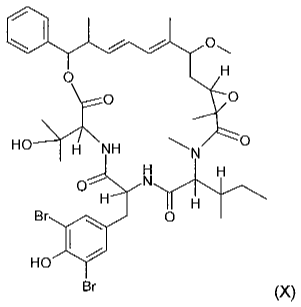

11. Pharmazeutische Zusammensetzung zur Verwendung bei der Behandlung und/oder Prophylaxe von Krebserkrankungen, die mindestens eine Verbindung der Formel (I) nach den Ansprüchen 1 bis 10 enthält.

12. Verbindung der Formel (I), 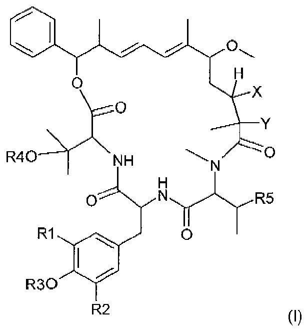 worin
X und Y unabhängig voneinander OH, O-(C₁-C₆)-Alkyl, NH₂ oder NH-(C₁-C₆)-Alkyl bedeuten, oder X und Y zusammen eine Gruppe -O- bilden oder wobei X und Y zusammen eine weitere Bindung zwischen den C-Atomen, an die sie gebunden sind, bilden,
R1 und R2 unabhängig voneinander H, Cl oder Br bedeuten,
R3 H, (C₁-C₆)-Alkyl, C(=O)-(C₁-C₆)-Alkyl oder (C₁-C₆)-Alkylen-NH-(C₁-C₆)-alkyl bedeutet,
R4 H, (C₁-C₆)-Alkyl oder C(=O)-(C₁-C₆)-Alkyl bedeutet, und
R5 Methyl oder Ethyl bedeutet,
mit der Maßgabe, dass dann, wenn
X und Y unabhängig voneinander OH, O-(C₁-C₆)-Alkyl, NH₂ oder NH-(C₁-C₆)-Alkyl bedeuten, oder X und Y zusammen eine Gruppe -O- bilden,
R1 und R2 unabhängig voneinander H oder Cl bedeuten,
R3 H, (C₁-C₆)-Alkyl, C(=O)-(C₁-C₆)-Alkyl oder (C₁-C₆)-Alkylen-NH-(C₁-C₆)-alkyl bedeutet, und
R4 H, (C₁-C₆)-Alkyl oder C(=O)-(C₁-C₆)-Alkyl bedeutet,
R5 nicht Ethyl bedeuten kann,
oder ein physiologisch verträgliches Salz einer Verbindung der Formel (I).

13. Verbindung der Formel (I) nach Anspruch 12, wobei es sich bei der Verbindung der Formel (I) um eine Verbindung der Formeln (V) bis (VIII) handelt.

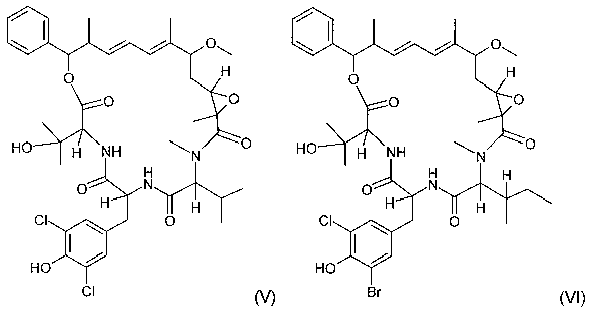

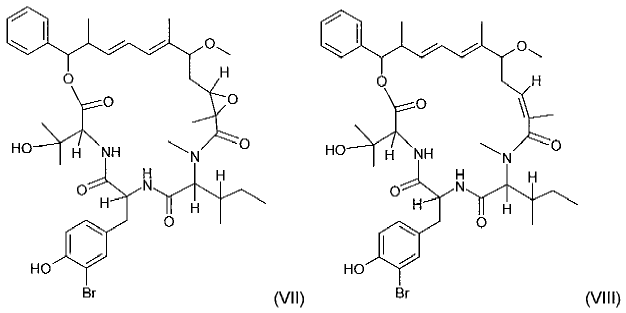

14. Verfahren zur Herstellung einer Verbindung der Formel (I) nach Anspruch 12 oder 13 oder eines physiologisch verträglichen Salzes davon, bei dem man
1. den Stamm *Nannocystis sp.* ST 201196 (DSM 18870) oder eine seiner Varianten und/oder Mutanten unter geeigneten Bedingungen in einem Kulturmedium, das eine Cl- und/oder Br-Quelle enthält, fermentiert, bis sich eine oder mehrere der Verbindungen der Formel (I) in dem Kulturmedium anhäuft, und
2. eine Verbindung der Formel (I) aus dem Kulturmedium isoliert, und
3. gegebenenfalls die Verbindung der Formel (I) derivatisiert und/oder in ein physiologisch verträgliches Salz umwandelt.

15. Verfahren nach Anspruch 14, bei dem in dem Kulturmedium mindestens eine Br-Quelle vorliegt.

16. Pharmazeutische Zusammensetzung, die mindestens eine Verbindung der Formel (I) nach Anspruch 12 oder 13 enthält.

## Revendications

1. Composé de formule (I), 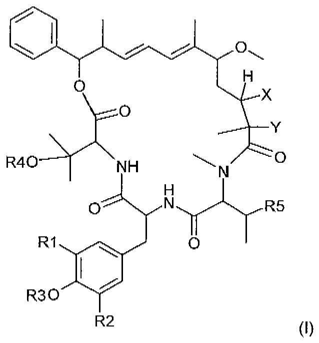 dans laquelle
X et Y indépendamment l'un de l'autre sont OH, O-(C₁-C₆)-alkyle, NH₂ ou NH-(C₁-C₆)-alkyle, ou X et Y forment ensemble un groupe -O- ou dans laquelle X et Y forment ensemble une liaison supplémentaire entre les atomes de C auxquels ils sont liés,
R1 et R2 indépendamment l'un de l'autre sont H, CI or Br,
R3 est H, (C₁-C₆)-alkyle, C(=O)-(C₁-C₆)-alkyle ou (C₁-C₆)-alkylène-NH-(C₁-C₆)-alkyle,
R4 est H, (C₁-C₆)-alkyle ou C(=O)-(C₁-C₆)-alkyle, et
R5 est méthyle ou éthyle,
ou un sel physiologiquement tolérable d'un composé de formule (I),
destiné à être utilisé dans le traitement et/ou la prophylaxie des maladies cancéreuses.

2. Composé de formule (I) destiné à être utilisé selon la revendication 1, dans laquelle X et Y forment ensemble un groupe -O- ou dans laquelle X et Y forment ensemble une liaison supplémentaire entre les atomes de C auxquels ils sont liés.

3. Composé de formule (I) destiné à être utilisé selon la revendication 1 ou 2, dans laquelle au moins l'un de R1 et R2 est Cl ou Br.

4. Composé de formule (I) destiné à être utilisé selon les revendications 1 à 3, dans laquelle R3 est H ou (C₁-C₆)-alkyle.

5. Composé de formule (I) destiné à être utilisé selon les revendications 1 à 4, dans laquelle R4 est H.

6. Composé de formule (I) destiné à être utilisé selon les revendications 1 à 4, dans laquelle R5 est éthyle.

7. Composé de formule (I) destiné à être utilisé selon la revendication 1, dans laquelle
X et Y forment ensemble un groupe -O-, ou X et Y forment une double liaison supplémentaire entre les atomes de C auxquels ils sont liés,
R1 et R2 indépendamment l'un de l'autre sont H, Cl ou Br,
R3 et R4 indépendamment l'un de l'autre sont H, (C₁-C₆)-alkyle ou C(=O)-(C₁-C₆)-alkyle, et
R5 est méthyle ou éthyle.

8. Composé de formule (I) destiné à être utilisé selon la revendication 1, dans laquelle
X et Y forment ensemble un groupe -O-, ou X et Y forment une double liaison supplémentaire entre les atomes de C auxquels ils sont liés,
R1 et R2 indépendamment l'un de l'autre sont H, Cl ou Br,
R3 est H ou (C₁-C₆)-alkyle,
R4 est H, et
R5 est méthyle ou éthyle.

9. Composé de formule (I) destiné à être utilisé selon la revendication 1, dans laquelle
X et Y forment ensemble un groupe -O-, ou X et Y forment une double liaison supplémentaire entre les atomes de C auxquels ils sont liés,
R1 et R2 sont tous deux CI,
R3 est H ou (C₁-C₆)-alkyle,
R4 est H, et
R5 est méthyle ou éthyle.

10. Composé de formule (I) destiné à être utilisé selon la revendication 1, le composé de formule (I) étant un composé des formules (II) à (X)

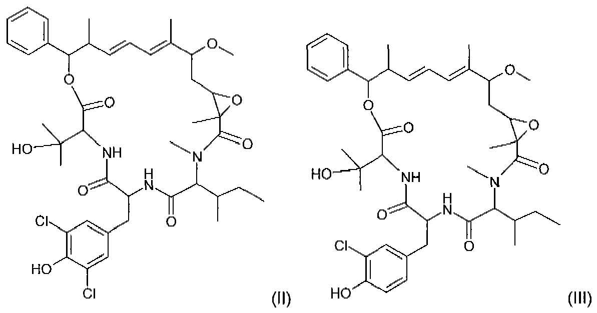

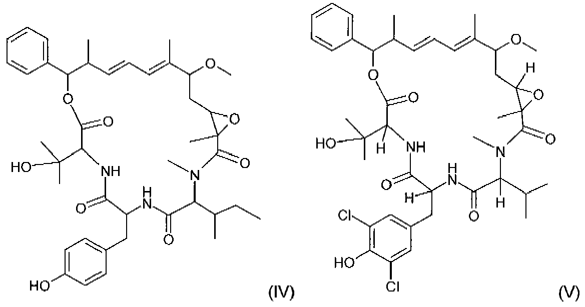

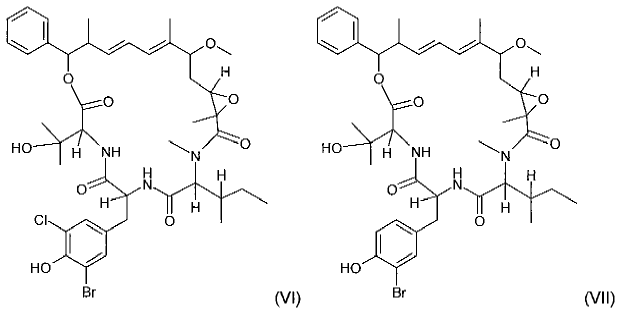

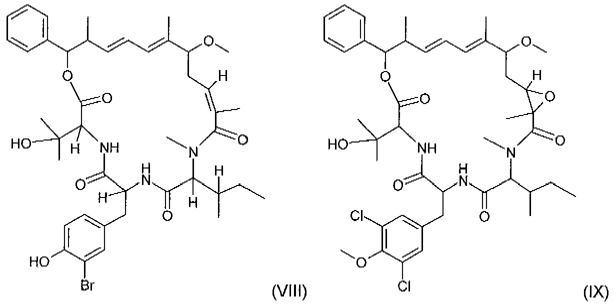

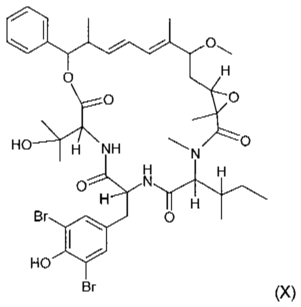

11. Composition pharmaceutique destinée à être utilisée dans le traitement et/ou la prophylaxie des maladies cancéreuses, contenant au moins un composé de formule (I) selon les revendications 1 à 10.

12. Composé de formule (I), 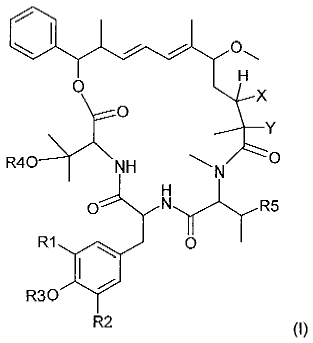 dans laquelle
X et Y indépendamment l'un de l'autre sont OH, O-(C₁-C₆)-alkyle, NH₂ ou NH-(C₁-C₆)-alkyle, ou X et Y forment ensemble un groupe -O- ou dans laquelle X et Y forment ensemble une liaison supplémentaire entre les atomes de C auxquels ils sont liés,
R1 et R2 indépendamment l'un de l'autre sont H, CI or Br,
R3 est H, (C₁-C₆)-alkyle, C(=O)-(C₁-C₆)-alkyle ou (C₁-C₆)-alkylène-NH-(C₁-C₆)-alkyle,
R4 est H, (C₁-C₆)-alkyle ou C(=O)-(C₁-C₆)-alkyle, et
R5 est méthyle ou éthyle,
à condition que lorsque
X et Y indépendamment l'un de l'autre sont OH, O-(C₁-C₆)-alkyle, NH₂ ou NH-(C₁-C₆)-alkyle, ou X et Y forment ensemble un groupe -O-,
R1 et R2 indépendamment l'un de l'autre sont H ou Cl,
R3 est H, (C₁-C₆)-alkyle, C(=O)-(C₁-C₆)-alkyle ou (C₁-C₆)-alkylène-NH-(C₁-C₆)-alkyle, et
R4 est H, (C₁-C₆)-alkyle ou C(=O)-(C₁-C₆)-alkyle,
R5 ne puisse pas être éthyle,
ou un sel physiologiquement tolérable d'un composé de formule (I).

13. Composé de formule (I) selon la revendication 12, le composé de formule (I) étant un composé de formules (V) à (VIII).

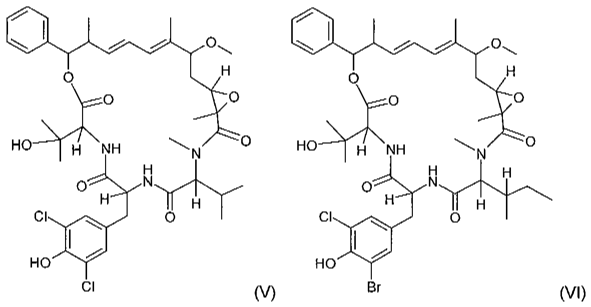

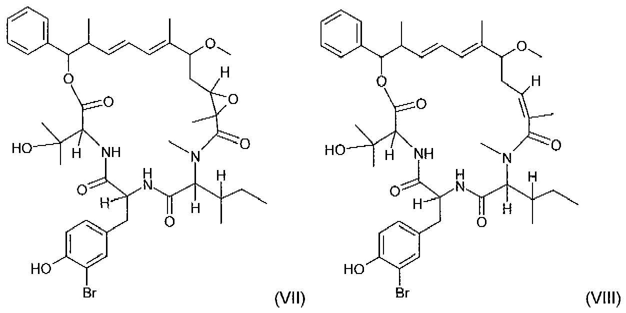

14. Procédé de préparation d'un composé de formule (I) selon la revendication 12 ou 13 ou d'un sel physiologiquement tolérable de celui-ci, qui comprend
1. la fermentation de la souche *Nannocystis sp.* ST 201196 (DSM 18870) ou une de ses variantes et/ou mutants dans des conditions convenables dans un milieu de culture qui contient une source de CI et/ou de Br, jusqu'à l'accumulation d'un ou plusieurs des composés de formule (I) dans le milieu de culture, et
2. l'isolement d'un composé de formule (I) du milieu de culture, et
3. éventuellement la dérivatisation du composé de formule (I) et/ou sa transformation en un sel physiologiquement tolérable.

15. Procédé selon la revendication 14, dans lequel au moins une source de Br est présente dans le milieu de culture.

16. Composition pharmaceutique, contenant au moins un composé de formule (I) selon la revendication 12 ou 13.
